# EUROPÄISCHE PATENTSCHRIFT

(11) **EP 1 557 189 B1**
(45) Veröffentlichungstag und Bekanntmachung des Hinweises auf die Patenterteilung: **17.05.2006**
(21) Anmeldenummer: 05007617.3
(22) Anmeldetag: 13.01.2000
(51) Int. Cl.: A61M 5/20, A61M 5/315

(54) **Injektionsgerät**
Injection device
Appareil d'injection

(30) Priorität: 14.01.1999 DE 29900482 U; 04.05.1999 DE 29907881 U
(43) Veröffentlichungstag der Anmeldung: 27.07.2005
(62) Teilanmeldung aus: 00906193.8
(73) Patentinhaber: Haselmeier Sàrl, 1295 Mies (VD) (CH)
(72) Erfinder: Hörl, Jürgen, 74369 Löchgau (DE); Bechtold, Herbert, 78056 Villingen-Schwenningen (DE); Hambrecht, Gerhard, 74749 Rosenberg (DE)
(74) Vertreter: Raible, Tobias

(56) Entgegenhaltungen:
- DE-A1- 4 223 958
- DE-A1- 19 519 147
- US-A- 5 514 097

## Beschreibung

Die Erfindung betrifft ein Injektionsgerät mit einem Behälter zur Aufnahme einer Injektionsflüssigkeit enthaltenden Kartusche, an deren proximalen Ende eine Injektionsnadel befestigbar ist.

Ein derartiges Injektionsgerät ist bekannt aus der DE 42 23 958 A1. Das dort dargestellte und beschriebene Injektionsgerät arbeitet sehr zuverlässig und präzise, ist aber weniger gut geeignet zur Verwendung größerer Kartuschen mit größeren Mengen von Injektionsflüssigkeit.

Es ist deshalb eine Aufgabe der Erfindung, ein neues Injektionsgerät bereitzustellen.

Eine Lösung dieser Aufgabe ergibt sich durch den Gegenstand des Anspruchs 1. Das im Anspruch 1 angegebene Prinzip ist besonders "narrensicher", wenn es darum geht, eine verbrauchte Kartusche durch eine neue zu ersetzen.

Weitere Einzelheiten und vorteilhafte Weiterbildungen der Erfindung ergeben sich aus den im folgenden beschriebenen und in der Zeichnung dargestellten, in keiner Weise als Einschränkung der Erfindung zu verstehenden Ausführungsbeispielen, sowie aus den Unteransprüchen. Es zeigt:
- Fig. 1: eine raumbildliche Darstellung eines erfindungsgemäßen Injektionsgeräts, als Übersichtsdarstellung,
- Fig. 2: eine Seitenansicht des Injektionsgeräts der Fig. 1, bei dem die Spannkappe 56 abgeschraubt und neben dem Gerät dargestellt ist,
- Fig. 2A: eine Abwicklung einer beim erfindungsgemäßen Injektionsgerät verwendbaren Skala, in schematisierter Darstellung,
- Fig. 3: eine Darstellung analog Fig. 1, wobei ein proximaler Abschnitt des Gehäuses im Schnitt dargestellt ist,
- Fig. 4: eine Darstellung des Injektionsgeräts nach einer Injektion, wobei der proximale Teil im Längsschnitt dargestellt ist,
- Fig. 5: eine auseinandergezogene, raumbildliche Darstellung von Bestandteilen des proximalen Teils des Injektionsgeräts,
- Fig. 6: eine auseinandergezogene, raumbildliche Darstellung, welche verschiedene Bestandteile des mittleren Teils eines erfindungsgemäßen Geräts zeigt,
- Fig. 7: eine auseinandergezogene, raumbildliche Darstellung analog Fig. 6, welche ebenfalls Teile eines erfindungsgemäßen Geräts zeigt,
- Fig. 8: eine raumbildliche, vergrößerte Darstellung einer bevorzugten Form eines Stößels, wie er bei der vorliegenden Erfindung Verwendung finden kann,
- Fig. 9: eine auseinandergezogene, raumbildliche Darstellung von Bestandteilen des distalen Teils des Injektionsgeräts, in einer Darstellung analog Fig. 6 und 7, aber in einem größeren Maßstab,
- Fig. 10: einen Längsschnitt durch ein Teil, welches u.a. einen Clip bildet, der als Auslöser bei einer Injektion dient,
- Fig. 11: einen Längsschnitt durch den Einstellknopf eines erfindungsgemäßen Injektionsgeräts,
- Fig. 12: eine Seitenansicht eines Bestandteils einer Zustellhülse, wie sie bei einem erfindungsgemäßen Injektionsgerät für die Dosiseinstellung Verwendung findet,
- Fig. 13: eine stark vergrößerte Darstellung der Teile einer Zustellhülse, wie sie bei einem erfindungsgemäßen Injektionsgerät für die Dosiseinstellung Verwendung findet,
- Fig. 14: eine raumbildliche Darstellung eines vorderen und eines hinteren Adapterteils, in einer gegenüber Fig. 6 vergrößerten Darstellung,
- Fig. 15: einen Längsschnitt durch verschiedene Teile, die im Gehäuse des Injektionsgeräts angeordnet sind, zur Erläuterung von deren funktionellem Zusammenwirken,
- Fig. 16: eine Darstellung eines erfindungsgemäßen Injektionsgeräts, bei welchem die Spannkappe 56 eingeschraubt ist, aber der Patient vergessen hat, eine Injektionsnadel einzusetzen; das Gerät kann nicht gespannt werden,
- Fig. 17: eine vergrößerte Darstellung der Einzelheit XVII der Fig. 16,
- Fig. 18: eine Darstellung analog Fig. 15, bei der verschiedene Kupplungen K1 bis K10 hervorgehoben sind, welche in ihrem funktionellen Zusammenwirken zur Arbeitsweise des erfindungsgemäßen Injektionsgeräts beitragen,
- Fig. 19: einen Längsschnitt durch ein erfindungsgemäßes Injektionsgerät in seiner gespannten Stellung, also der Stellung gemäß Fig. 1 und 3,
- Fig. 20: einen Schnitt, gesehen längs der Linie XX-XX der Fig. 19,
- Fig. 21: einen Schnitt, gesehen längs der Linie XXI-XXI der Fig. 19,
- Fig. 22: einen Längsschnitt durch ein erfindungsgemäßes Injektionsgerät in seiner gespannten Stellung und nach Abschrauben der Spannkappe diese Stellung entspricht der Stellung der Fig. 2,
- Fig. 23: einen Längsschnitt analog Fig. 22, wobei aber eine Injektionsdosis eingestellt wurde,
- Fig. 24: einen Längsschnitt durch ein erfindungsgemäßes Injektionsgerät während der ersten Phase einer Injektion (Einstich der Nadel, aber vor dem Auspressen von Injektionsflüssigkeit),
- Fig. 25: einen Längsschnitt analog Fig. 24, aber während der zweiten Phase einer Injektion (Auspressen von Injektionsflüssigkeit nach Einstechen der Nadel),
- Fig. 26: einen Längsschnitt, welcher den Beginn eines Kartuschenwechsels zeigt,
- Fig. 27: eine Darstellung, welche - im Anschluss an Fig. 26 - eine weitere Phase des Kartuschenwechsels zeigt,
- Fig. 28: eine Darstellung, welche eine an Fig. 27 anschließende Phase des Kartuschenwechsels zeigt,
- Fig. 29: eine vergrößerte Darstellung der Einzelheit XXIX der Fig. 28, welche das Verrasten des Stößels 108 in seiner distalen Endlage zeigt,
- Fig. 30: eine schematische Darstellung, welche zeigt, wie eine verbrauchte Kartusche 52 dem Kartuschenhalter 80 entnommen wird,
- Fig. 31: eine Darstellung, welche zeigt, wie eine neue Kartusche in den Kartuschenhalter 80 eingesetzt wird,
- Fig. 32: eine Darstellung, welche zeigt, wie der neu geladene Kartuschenhalter (gemäß Fig. 31) an das Injektionsgerät angeschraubt wird,
- Fig. 33: eine Darstellung, welche die an Fig. 32 anschließende Phase zeigt, nämlich das Anschrauben des proximalen Gehäuseteils und die dabei ablaufenden Vorgänge,
- Fig. 34: eine Darstellung einer Variante, bei der im Vergleich zu Fig. 1 als Sichtfenster eine Vielzahl runder Löcher 54A verwendet wird,
- Fig. 35: eine Draufsicht, gesehen in Richtung des Pfeils XXXV der Fig. 34, aber in einem relativ zu Fig. 34 vergrößerten Maßstab,
- Fig. 36: eine schematische Darstellung der axialen Verzahnung 220 der Zustellhülse 151 und des Zusammenwirkens dieser Verzahnung mit einem Rastglied 184 bei der Dosiseinstellung vor einer Injektion,
- Fig. 37: eine raumbildliche, auseinandergezogene Darstellung von Teilen, die beim Kartuschenwechsel eine Rolle spielen, und
- Fig. 38 bis 40: eine synoptische Darstellung zur Erläuterung der Wirkungsweise der Kupplungen K4 und K5 bei verschiedenen Betriebszuständen eines Injektionsgeräts nach der Erfindung.

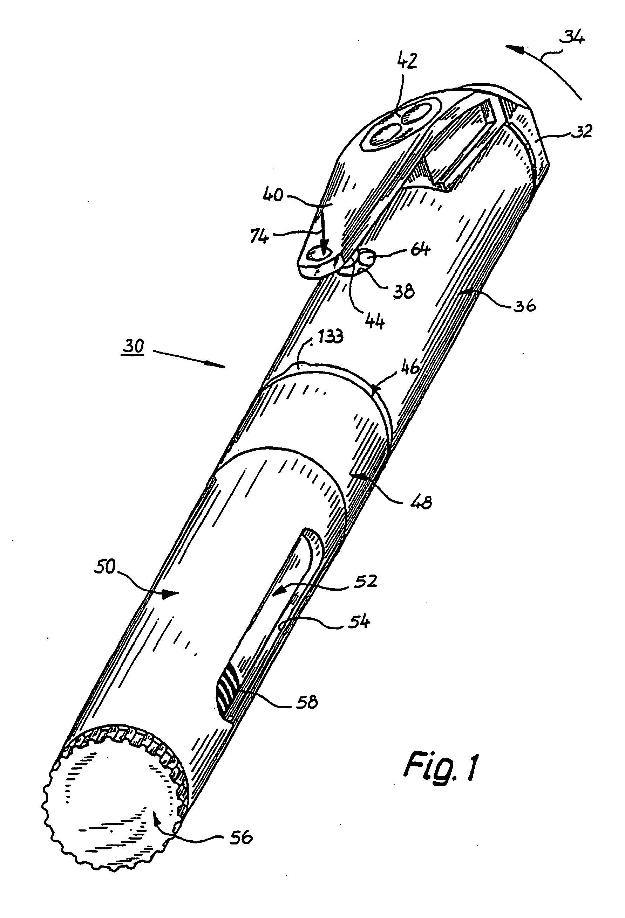

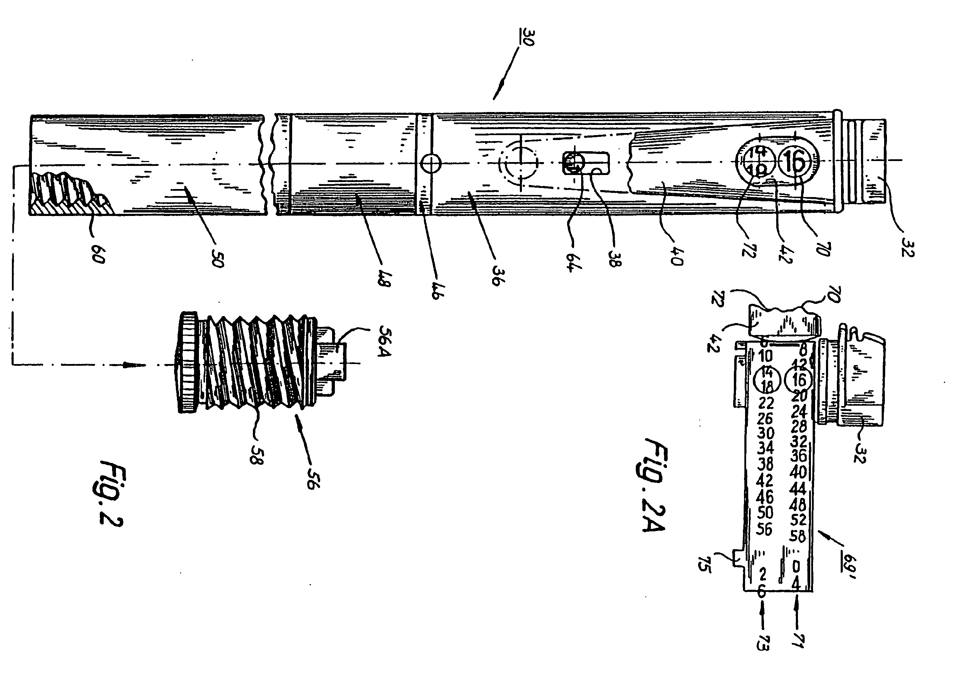

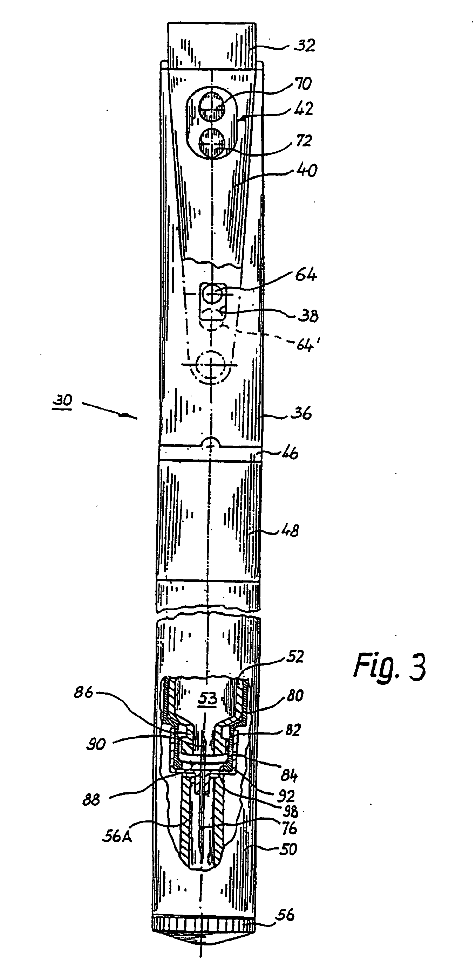

In der nachfolgenden Beschreibung werden die Begriffe proximal und distal in der in der Medizin üblichen Weise verwendet:
- Proximal: = die dem Patienten zugewandte Seite, also in Fig. 3 die untere Seite des Injektionsgeräts mit der Nadel.
- Distal: = die vom Patienten abgewandte Seite, also in Fig. 1 und 2 die obere Seite.

**Fig.1** 1 zeigt in schematisierter Form eine raumbildliche Darstellung eines erfindungsgemäßen Injektionsgeräts 30. Dieses hat an seinem distalen Ende einen Einstellknopf 32, der durch Drehen in Richtung eines Pfeils 34 eine Dosiseinstellung ermöglicht, sofern sich das Gerät in seiner Stellung gemäß Fig. 2 befindet. (In der Stellung gemäß Fig. 1 ist die Dosiseinstellung nicht aktiviert.) Der Knopf 32 ist drehbar angeordnet in einem rohrförmigen distalen Gehäuseteil 36, in dem sich eine längliche Rastöffnung 38 befindet und an dem ein federnder Clip 40 befestigt ist. Im Clip 40 befindet sich eine Lupe 42 zum Ablesen der eingestellten Dosis. Der Clip 40 hat einen radial nach innen ragenden Vorsprung 44, der zur Auslösung einer Injektion dient und der länglichen Rastöffnung 38 gegenüberliegt.

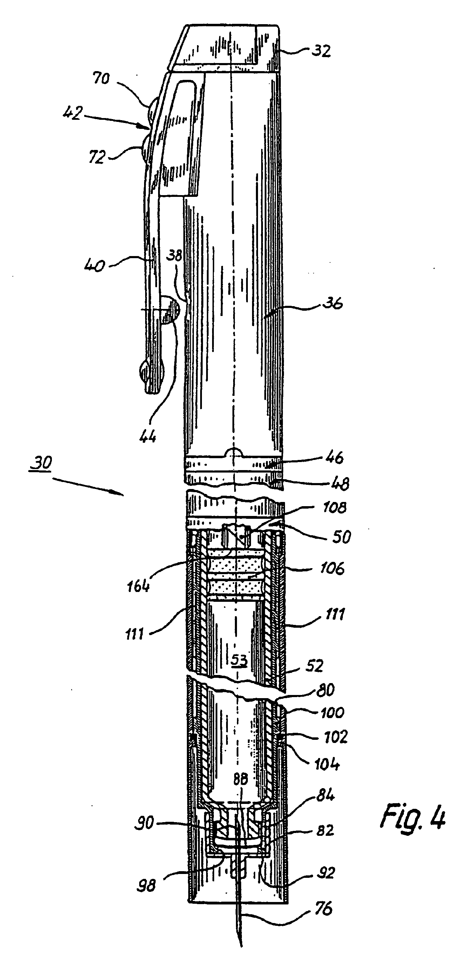

An das distale Gehäuseteil 36 schließt sich in proximaler Richtung ein ringförmiges Teil 46 an, das mit dem Gehäuseteil 36 fest verbunden ist. Auf dieses folgt in proximaler Richtung ein mittleres Gehäuseteil 48. Hieran schließt sich in proximaler Richtung ein proximales Gehäuseteil 50 an, welches eine Kartusche 52 mit einer zu injizierenden Flüssigkeit 53 (Fig. 4) aufnimmt und mit wenigstens einem Sichtfenster 54 versehen ist, durch das der Füllzustand der Kartusche 52 beobachtet werden kann.

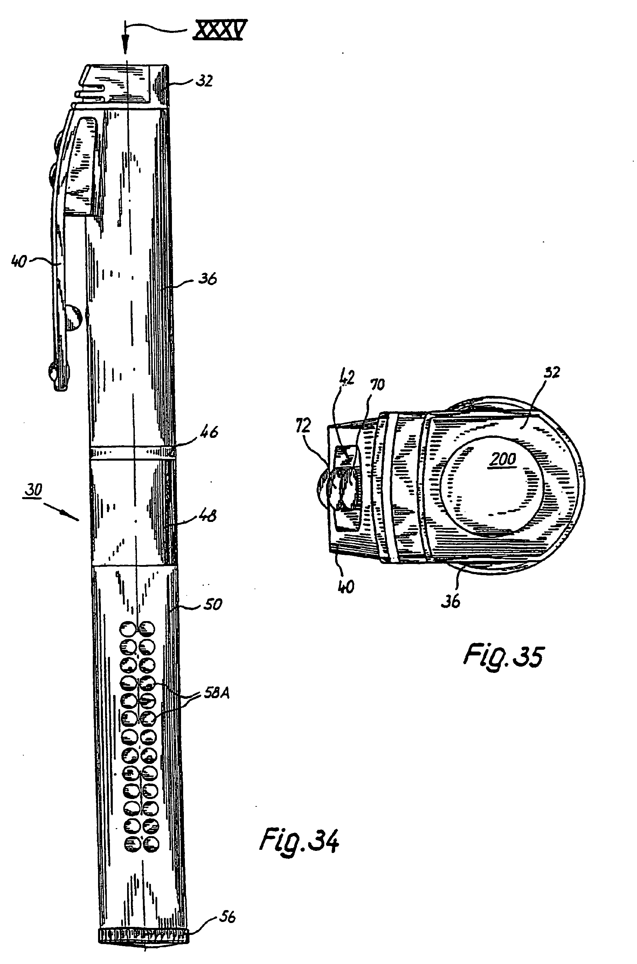

Mit Vorteil wird gemäß Fig. 34 als Sichtfenster eine Vielzahl kleiner Durchbrechungen 54A verwendet. Dies hat den Vorteil, dass der Patient nicht mit seinen Fingem durch das Fenster 54 durchgreifen und dadurch die Bewegung der Kartusche 52 beim Einstich bremsen kann, dass aber die visuelle Beobachtung des Füllzustands des Kartusche 52 sehr gut möglich ist.

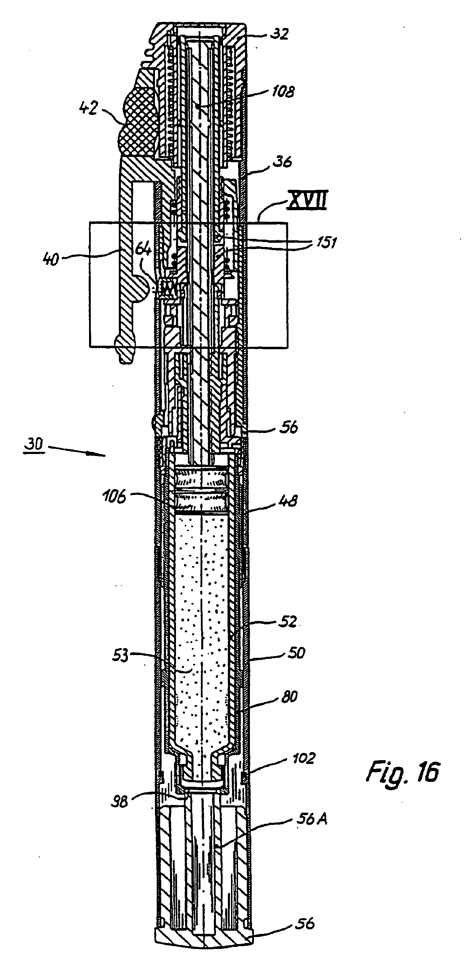

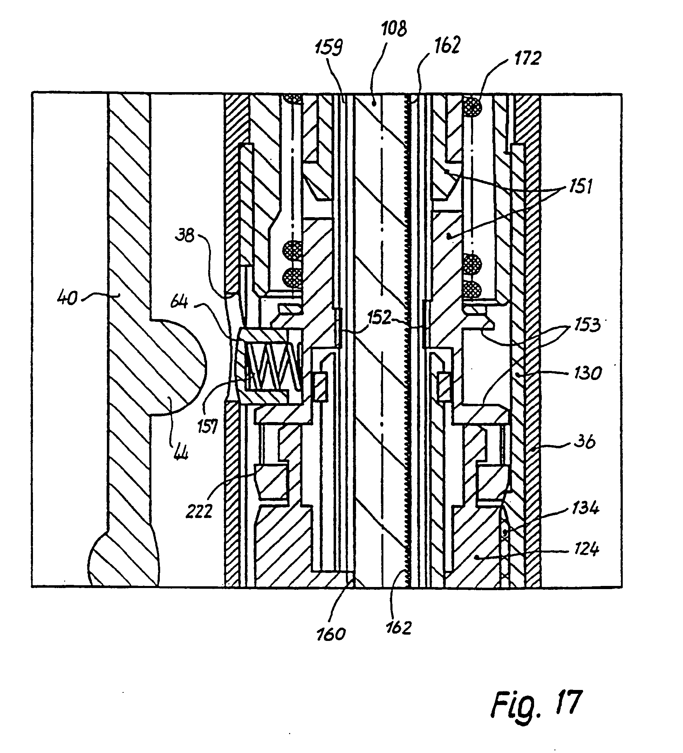

Am proximalen Ende des Injektionsgeräts 30 befindet sich zum Spannen eine Spannkappe 56, die mit ihrem Außengewinde 58 in ein entsprechendes Innengewinde 60 (Fig. 2) des proximalen Gehäuseteils 50 eingeschraubt wird, um das Injektionsgerät vor einer Injektion zu spannen. Wie nachfolgend bei Fig. 16 und 17 beschrieben wird, ist das Spannen des Injektionsgeräts 30 nur möglich, falls eine Nadel 76 montiert ist. In der Stellung gemäß Fig. 1 ist also das Injektionsgerät 30 gespannt, da eine Nadel 76 montiert und die Spannkappe 56 bis zum Anschlag in das proximale Gehäuseteil 50 eingeschraubt ist. Dabei befindet sich ein federnder Rastzapfen 64 am distalen Ende der länglichen Rastöffnung 38, vgl. Fig. 3. In dieser Stellung befindet sich der Einstellknopf 32 im Leerlauf, d.h. man kann ihn in Richtung des Pfeiles 34 (Fig. 1) drehen, ohne dass eine Dosis eingestellt wird. Die Dosiseinstellung ist also hier deaktiviert.

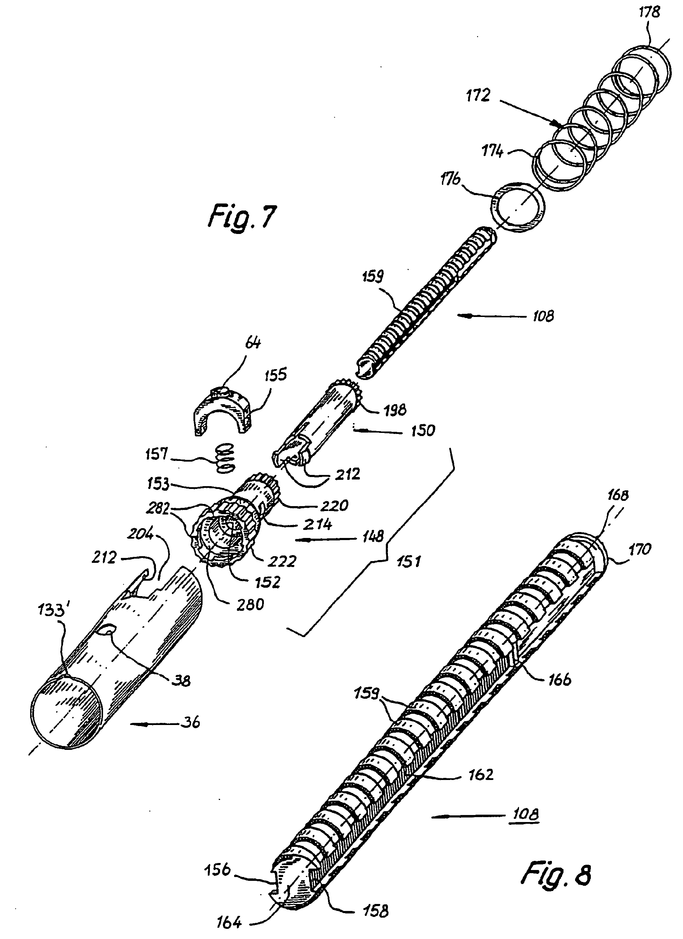

**Fig. 2** zeigt das Injektionsgerät 30 nach dem Spannen und nach dem Abschrauben der Spannkappe 56. Letztere wird vor einer Injektion abgeschraubt, um das Gerät für eine Injektion vorzubereiten. Dabei bewegt sich unter der Wirkung einer Spannfeder 172 (Fig. 7) der Rastzapfen 64 etwas in proximaler Richtung und legt sich gegen den proximalen Rand der länglichen Rastöffnung 38 an, vgl. Fig. 2. In dieser Stellung kann - vor einer Injektion - durch Drehen des Einstellknopfs 32 eine gewünschte Injektionsdosis eingestellt werden.

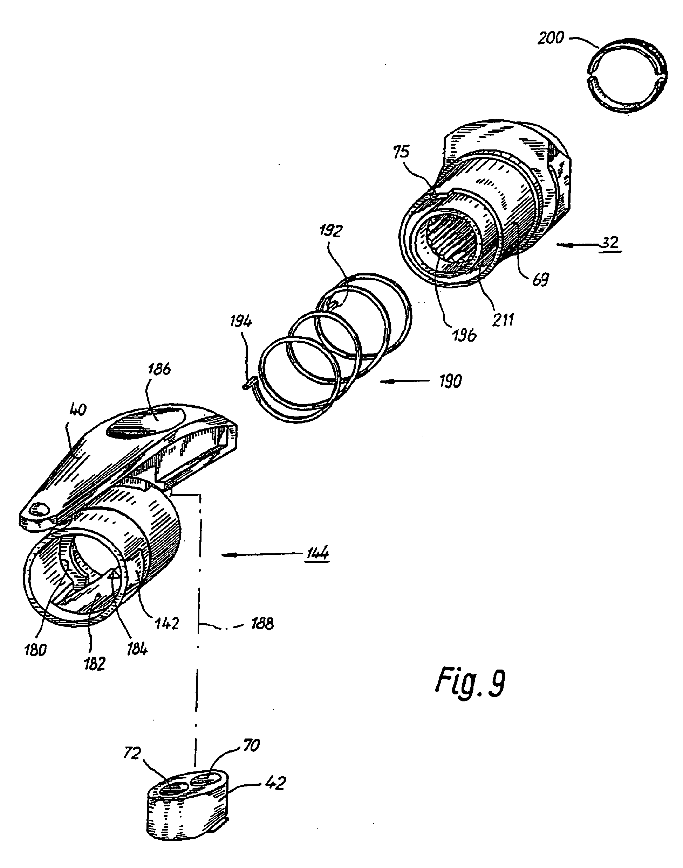

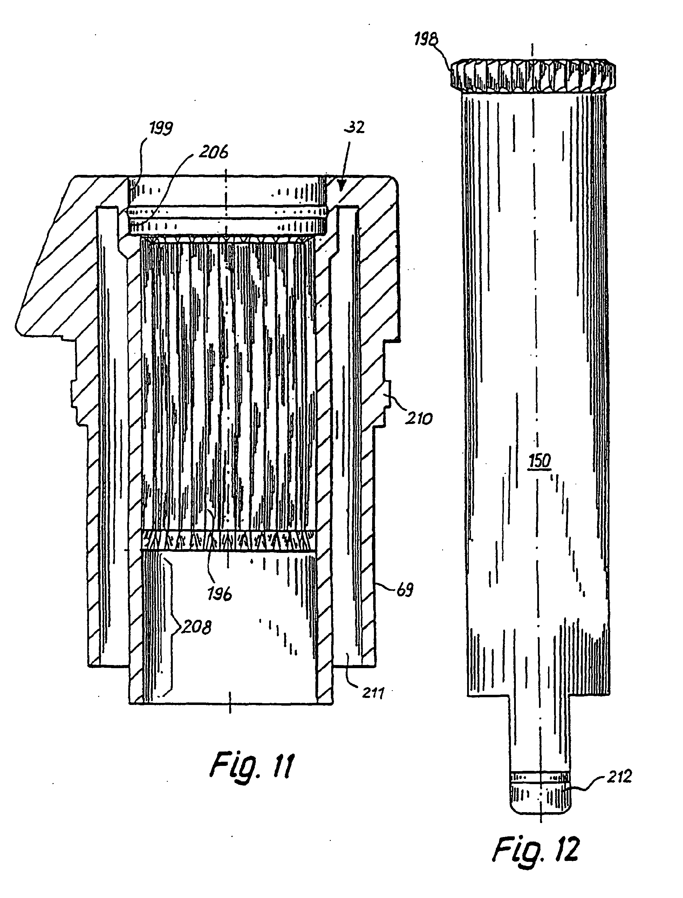

Zu ihrer Ablesung hat die Lupe 42 zwei Einzellupen 70, 72. Wegen der hohen Zahl der Dosiseinstellungen, die für eine Injektion möglich sind, im vorliegenden Fall z.B. dreißig verschiedene Einstellungen zwischen "0" und "58" Einheiten, ist die Skala zweireihig auf die Umfangsfläche 69 (Fig. 9 und 11) des Dosierknopfs 32 aufgedruckt. Fig. 2A zeigt eine Abwicklung 69' dieser Umfangsfläche 69. Die Skala hat eine erste Reihe 71 mit den Zahlen "0", "4", "8" etc., und eine zweite Reihe 73 mit den Zahlen "2", "6", "10", "14" etc. Ein Anschlag 75 markiert die Nullstellung. Die Linse 70 zeigt vergrößert eine Zahl aus der ersten Reihe 71, und die Linse 72 zeigt vergrößert eine Zahl aus der zweiten Reihe 73, vgl. Fig. 2. Dadurch ist eine unzweideutige Ablesung möglich, d.h. in Fig. 2 beträgt die eingestellte Dosis 16 Einheiten. (Beim vorliegenden Beispiel kann die Dosis immer um zwei Einheiten verstellt werden, ist also einstellbar von "0", "2", "4" etc. Einheiten bis °58° Einheiten.)

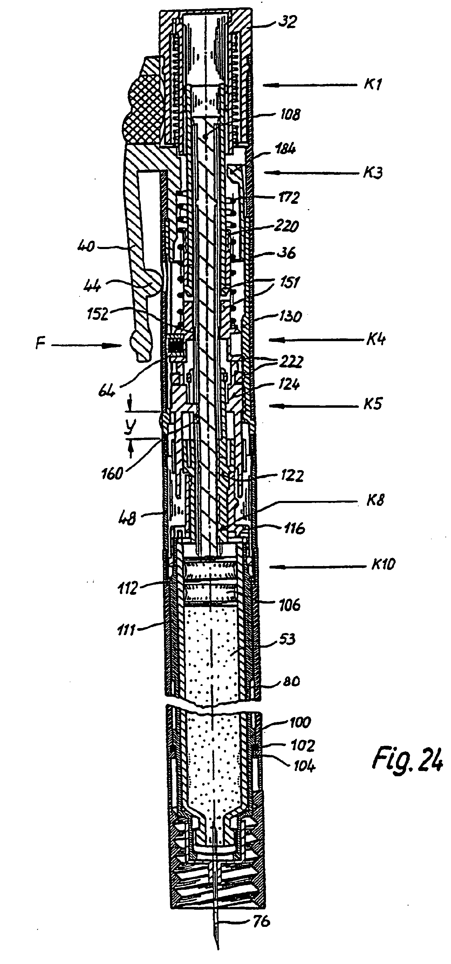

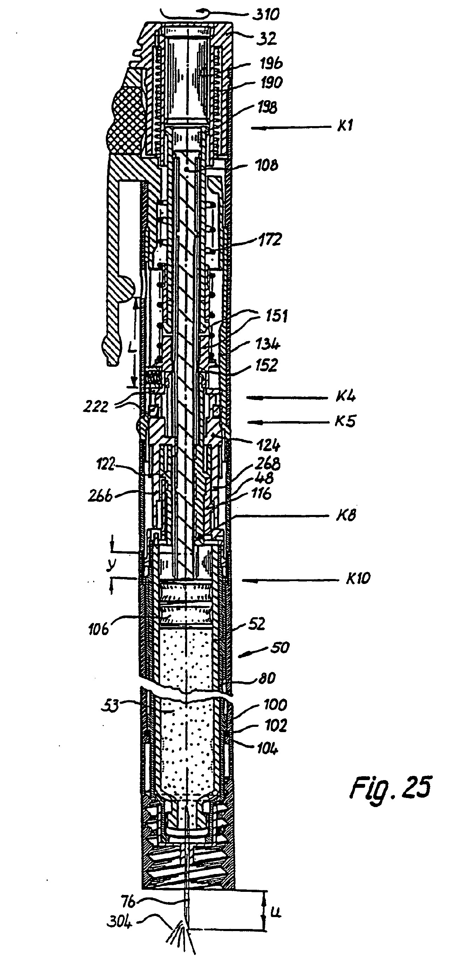

In der Stellung gemäß Fig. 2 ist auch eine Auslösung des Injektionsgeräts 30 möglich. Dies geschieht durch Druck auf den Clip 40 in Richtung eines Kraftvektors 74 (Fig. 1). Dabei wird durch den radial nach innen ragenden Vorsprung 44 der Rastzapfen 64 radial nach innen gedrückt. Die Spannfeder 172 (Fig. 7) bewirkt dann zuerst ein Einstechen der Injektionsnadel 76 (Fig. 4), und anschließend eine Injektion der eingestellten Injektionsdosis durch die eingestochene Nadel 76. Dies wird nachfolgend anhand der Fig. 24 und 25 beschrieben. Es handelt sich also um ein Gerät mit versteckter Nadel 76, d.h. diese ist für den Patienten nicht sichtbar, und der Injektionsvorgang läuft nach dem Auslösen vollautomatisch ab.

Das Gerät 30 wird also durch Einschrauben der Spannkappe 56 gespannt, und es wird durch Abschrauben der Spannkappe 56 in die Stellung gemäß Fig. 2 gebracht, in der die gewünschte Dosis eingestellt und anschließend injiziert werden kann.

**Fig. 3** zeigt das Injektionsgerät 30 in der Stellung gemäß Fig. 1, wobei der proximale Bereich aufgeschnitten dargestellt ist. Man erkennt die Kartusche 52 mit der in ihr enthaltenen Flüssigkeit 53. Die Kartusche 52 befindet sich in einem Kartuschenhalter 80, der an seinem proximalen Ende einen verjüngten Hals 82 hat und dort mit einem Außengewinde 84 versehen ist. Die Kartusche 52 ragt mit ihrem Hals 86 in diesen Hals 82. Sie ist an ihrem proximalen Ende mit einer Gummimembran 88 versehen, die bei Benutzung vom distalen Teil 90 der Nadel 76 durchstochen wird. Die Nadel 76, 90 ist an einem üblichen Nadelhalter 92 befestigt, der mit seinem Innengewinde auf das Außengewinde 84 des Kartuschenhalters 80 aufgeschraubt wird. Sie wird im gespannten Zustand durch die Spannkappe 56 geschützt und ist dann nicht sichtbar.

Wie Fig. 3 klar zeigt, liegt die Spannkappe 56 mit einem zylindrischen Abschnitt 56A gegen den Nadelhalter 92 an und beaufschlagt diesen in distaler Richtung. Dabei kommt der Rastknopf 64 in seine distale Stellung in der Ausnehmung 38, wie in Fig. 1 und 3 dargestellt, und der Einstellknopf 32 wird deaktiviert, wie bereits beschrieben.

Wenn der Patient vergessen hat, eine Nadel 76 anzuschrauben, fehlt der Nadelhalter 92, und das Gerät 30 kann nicht gespannt werden, weil der zylindrische Abschnitt 56A der Spannkappe 56 nun in den Hohlraum 98 des Kartuschenhalters 80 ragt, wie das Fig. 16 zeigt. Der Rastknopf 64 befindet sich dann in der Stellung gemäß Fig. 17, die in Fig. 3 mit 64' bezeichnet ist, d.h. ein Spannen und eine Injektion sind in diesem Zustand nicht möglich. So wird sichergestellt, dass das Injektionsgerät 30 ohne Nadel 76 nicht gespannt werden kann.

**Fig. 4** zeigt das Injektionsgerät 30 in dem Zustand nach einer Injektion. Die Nadel 76 ragt aus dem proximalen Gehäuseteil 50 heraus. Der Kartuschenhalter 80 hat einen Ringbund 100, und dieser liegt an gegen einen Dämpfungsring 102, der an einer Ringschulter 104 auf der Innenseite des proximalen Gehäuseteils 50 abgestützt ist. Dies ist die proximale Endlage des Kartuschenhalters 80.

In der Kartusche 52 befindet sich in der üblichen Weise ein Gummikolben 106, und dieser wird bei einer Injektion, nach dem Einstechen der Nadel 76, durch einen Stößel 108 in proximaler Richtung verschoben, um die zuvor mit dem Einstellknopf 32 eingestellte Flüssigkeitsmenge aus der Kartusche 52 auszupressen.

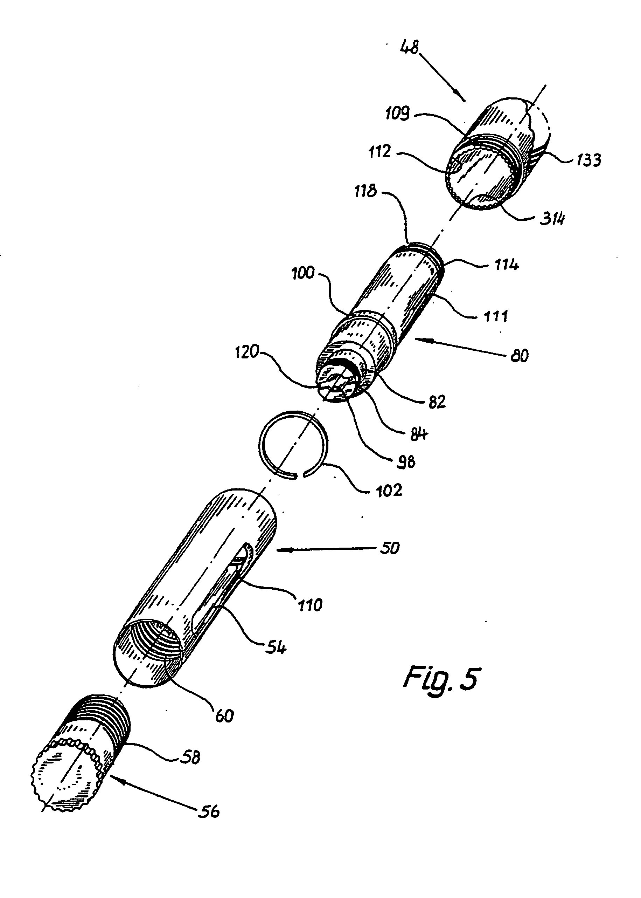

**Fig. 5** zeigt in auseinandergezogener, raumbildlicher Darstellung die verschiedenen Teile des proximalen Abschnitts des Injektionsgeräts 30. Im proximalen Gehäuseteil 50 ist das Innengewinde 60 vorgesehen, in welches das Außengewinde 58 der Spannkappe 56 geschraubt werden kann, um das Injektionsgerät zu spannen. Es handelt sich um ein Steilgewinde mit trapezförmigem Gewindequerschnitt.

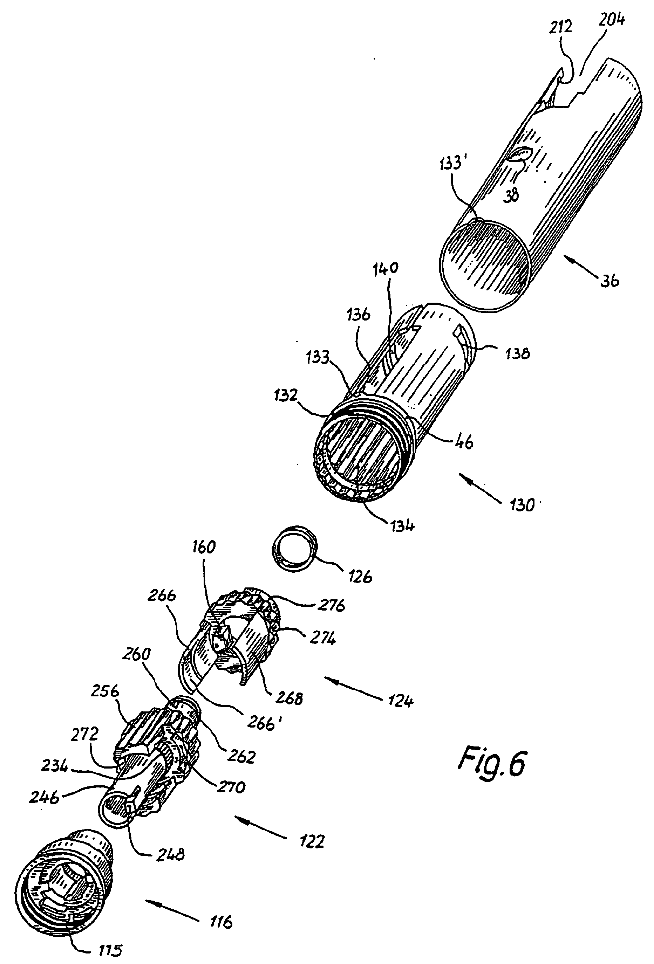

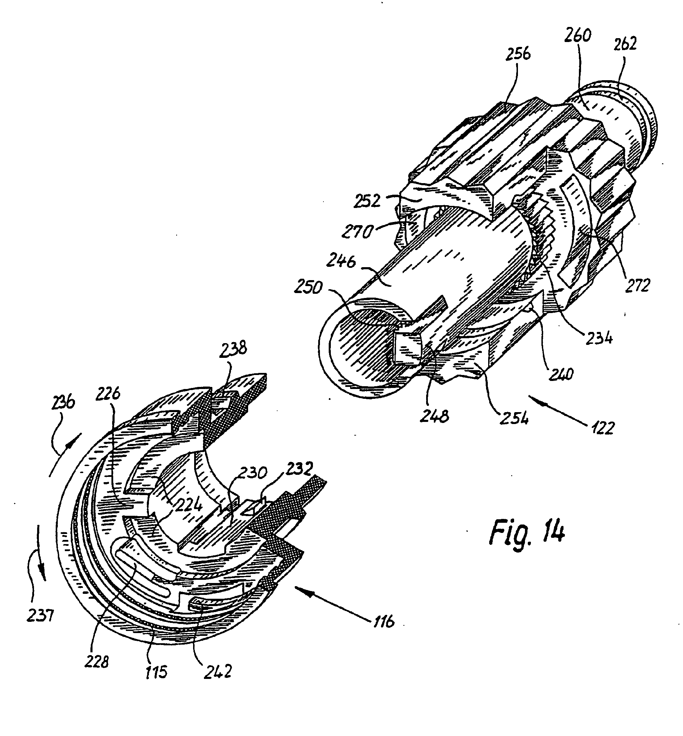

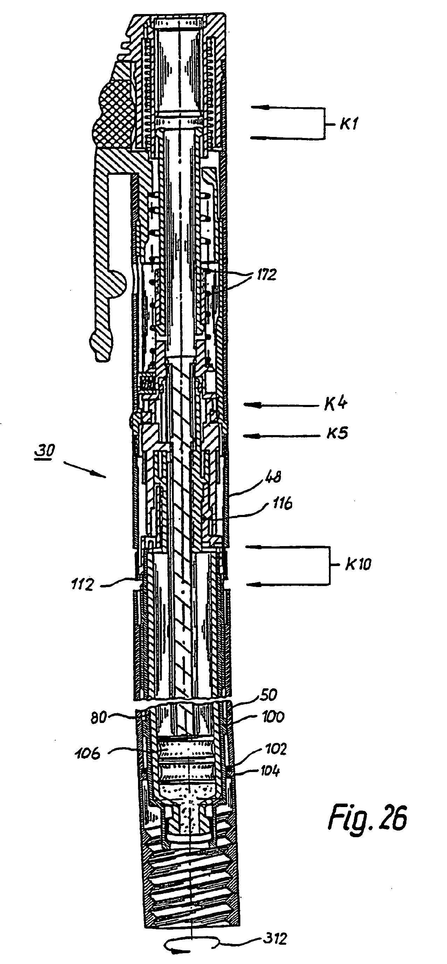

Das mittlere Gehäuseteil 48 hat an seinem proximalen Ende ein Außengewinde 109, das zur Verbindung mit einem Innengewinde 110 am distalen Ende des proximalen Gehäuseteils 50 dient. Auch hat das mittlere Gehäuseteil 48 an seinem proximalen Ende auf der Innenseite eine kurze axiale Längsverzahnung 112, die zur Längsführung einer Längsrippe 111 (oder mehrerer Längsrippen) auf der Außenseite des Kartuschenhalters 80 dient. Diese Längsrippen 111 bewirken eine Verdrehsicherung des Kartuschenhalters 80, solange sich das Injektionsgerät 30 in seinem betriebsbereiten Zustand befindet. Beim Wechsel einer Kartusche 52 ist diese Längsführung deaktiviert, d.h. dann gleiten die Längsrippen 111 aus der axialen Längsverzahnung 112 heraus, vgl. nachfolgend die Fig. 26 bis 32. Die Längsführung durch die Teile 111, 112 verhindert, dass sich der Kartuschenhalter 80 beim Wechsel einer Injektionsnadel 76 relativ zum Gehäuseteil 50 dreht und sich dadurch von einem vorderen Adapterteil 116 (Fig. 6 und 14) löst.

Der Kartuschenhalter 80 hat an seinem distalen Ende ein Außengewinde 114, das zur Verbindung mit einem Innengewinde 115 (Fig. 6 und 14) des vorderen Adapterteils 116 dient. Das Außengewinde 114 hat eine Unterbrechung 118, welche eine spezielle Funktion beim Kartuschenwechsel hat. Diese wird nachfolgend beschrieben.

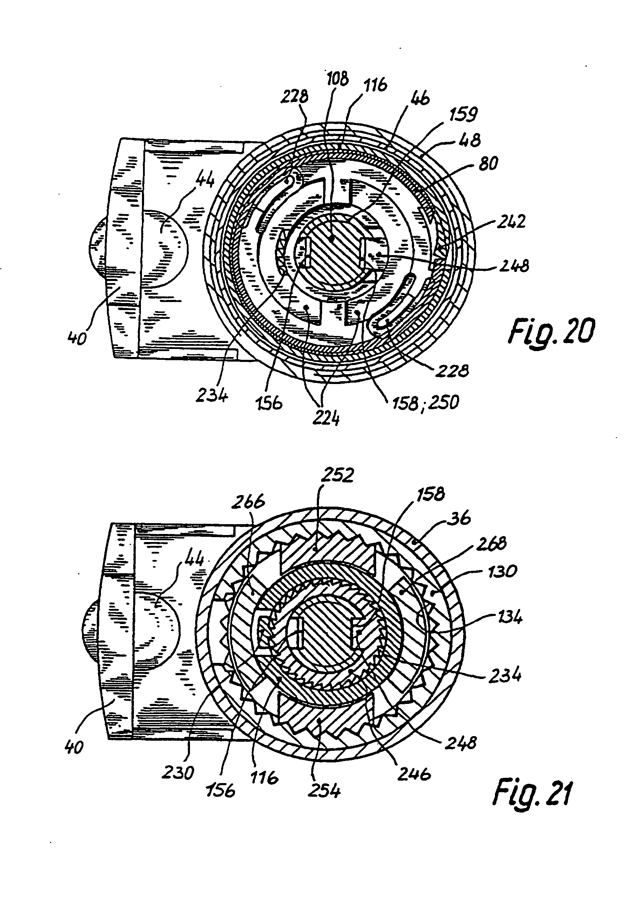

Der Hals 82 des Kartuschenhalters 80 ist nach Art eines Steckschlüssels ausgebildet und hat dazu eine diagonal verlaufende Nut 120, die eine Drehung des Adapterteils 116 (vgl. Fig. 6) ermöglicht, wenn man diesen Hals 82 in das Adapterteil 116 steckt, das entsprechend komplementär ausgebildet ist, damit es in die Nut 120 eingreifen kann, vgl. die Fig. 14 und 20. Die Teile 120 und 224 passen also wie Schlüssel und Schloss ineinander.

In Fig. 5 sind Nadel 76 und Kartusche 52 nicht dargestellt, um die Darstellung nicht mit zu vielen Einzelheiten zu überladen.

Die Fig. 6 bis 9 zeigen in auseinandergezogener, raumbildlicher Darstellung die restlichen Teile des Injektionsgeräts 30. Die Darstellung ist zum Teil stark schematisiert, um das Verständnis der Erfindung zu erleichtem. Fig. 9 ist im Vergleich zu Fig. 6 und 7 in einem vergrößerten Maßstab dargestellt, um die Einzelheiten besser darstellen zu können.

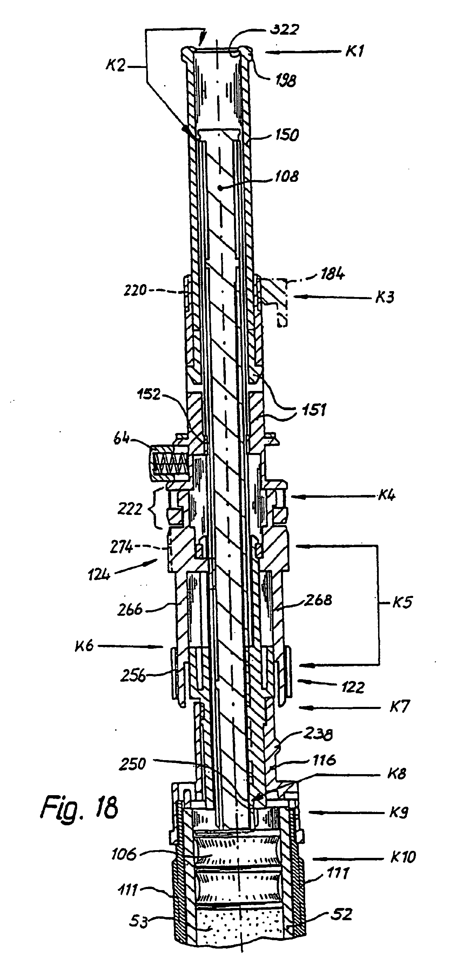

In **Fig. 6** folgt auf das vordere Adapterteil 116 mit seinem Innengewinde 115 ein hinteres Adapterteil 122, das bei der Montage mit dem vorderen, proximalen Adapterteil 116 drehbar, aber axial unverschiebbar verbunden wird, wobei zwischen den Teilen 116, 122 eine drehmomentabhängige Kupplung (K7 in Fig. 18) vorgesehen ist.

Es folgt ein Führungsteil 124, das zur axialen Führung des Stößels 108 (Fig. 7 & 8) dient, und das bei der Montage mit dem hinteren Adapterteil 122 drehfest, aber axial verschiebbar, verbunden wird. Darauf folgt ein Anschlagring 126, dessen Funktion nachfolgend erläutert wird und der in einer Ringnut 262 des hinteren Adapterteils 122 montiert wird.

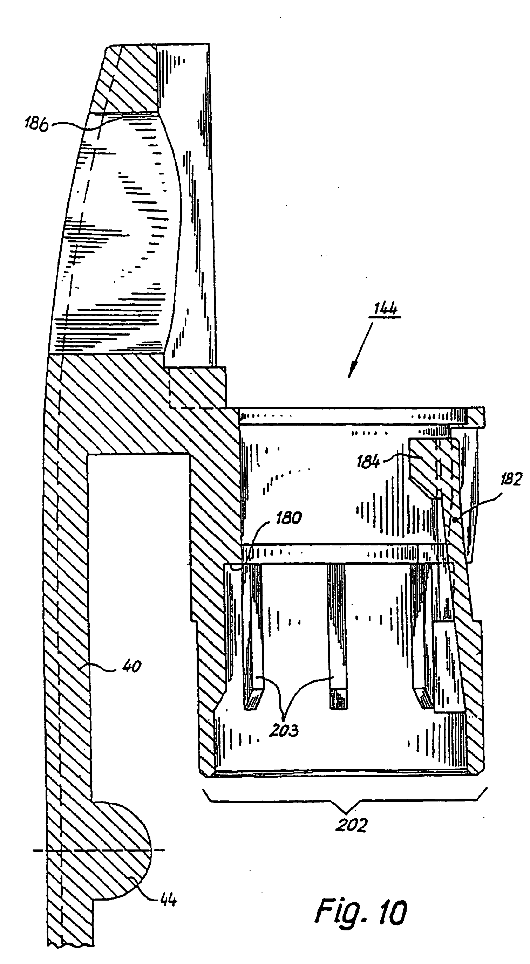

Das distale Gehäuseteil 36 nimmt ein Innenrohr 130 in der in Fig. 6 dargestellten Drehstellung auf, d.h. ein Längsschlitz 136 des Innenrohrs 130 fluchtet mit der Rastausnehmung 38 des Gehäuseteils 36. Das Innenrohr 130 bildet auf einem kurzen Teil seiner Längserstreckung das in Fig. 1 sichtbare ringförmige Teil 46, welches einen Vorsprung 133 als Verdrehsicherung hat, der in eine entsprechende Aussparung 133' des Gehäuseteils 36 ragt. An seinem proximalen Ende ist das Innenrohr 130 mit einem Außengewinde 132 versehen, das zur Verbindung mit einem Innengewinde 133 (Fig. 5) am distalen Ende des mittleren Gehäuseteils 48 dient. Das Innenrohr 130 ist auf einem Teil seiner Länge mit einer inneren Axialverzahnung 134 versehen. Seine axiale Längsnut 136 dient zur Längsführung des Rastglieds 64, damit sich dieses im Gehäuse 36 nicht verdrehen kann. Ferner hat Das Innenrohr 130 im Bereich seines distalen Endes zwei seitliche Rastausnehmungen 138, 140 zur Verrastung mit entsprechenden Widerhaken 142 an einem in Fig. 9 und 10 dargestellten Formstück 144, das u.a. den Clip 40 trägt.

**Fig. 7** zeigt rechts neben dem distalen Gehäuseteil 36 den vorderen Teil 148 und den hinteren Teil 150 einer sogenannten Zustellhülse 151, die zur Dosiseinstellung dient, also als Einstellglied. Die Teile 148 und 150 werden bei der Montage durch Einrasten fest miteinander verbunden und bilden dann die Zustellhülse 151. Diese wird bei der Dosiseinstellung verdreht und verschiebt dadurch den Stößel 108, der im montierten Zustand mit seinem Außengewinde 159 in ein Innengewinde 152 des Teils 148 eingreift, in proximaler Richtung.

Das Teil 148 führt in einer Ringnut 153 ein Trägerteil 155, welches den Rastzapfen 64 trägt und durch eine Druckfeder 157 radial nach außen gepresst wird, vgl. Fig. 7.

**Fig. 8** zeigt den Stößel 108 in vergrößertem Maßstab. Sein Außengewinde 159 ist ein Rechteck-Steilgewinde. Der Stößel 108 hat zwei Längsnuten 156, 158. In die Längsnut 156 greift im montierten Zustand ein als Eingriffsglied dienender Vorsprung 160 (Fig. 6) des Führungsteils 124 ein und verhindert dadurch bei der Dosiseinstellung und der Injektion eine Verdrehung des Stößels 108 relativ zum Gehäuse des Injektionsgeräts 30.

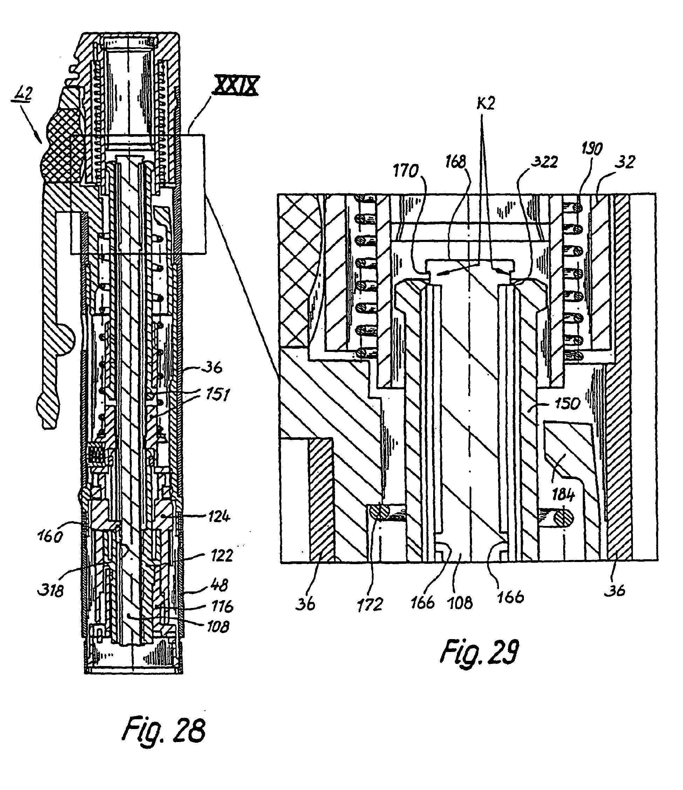

In der Längsnut 158 befindet sich, wie dargestellt, eine Mikroverzahnung 162 etwa wie bei einer Zahnstange. Die Verzahnung 162 erstreckt sich vom proximalen Ende 164 bis etwa zu einem Anschlag 166 in der Nut 158, hier etwa über zwei Drittel der Längserstreckung des Stößels 108. Im Bereich seines distalen Endes 168 ist der Stößel 108 mit einer als Rastelement dienenden Ringnut 170 versehen, welche beim Wechsel einer Kartusche 52 eine Funktion hat, die nachfolgend bei Fig. 29 näher erläutert wird.

In Fig. 7 ist mit 172 die Spannfeder bezeichnet, welche die Energie für eine Injektion speichert und mit ihrem proximalen Ende 174 im montierten Zustand über eine Gleitscheibe 176 am Teil 148 abgestützt ist und dieses in proximaler Richtung beaufschlagt.

Mit ihrem distalen Ende 178 ist die Feder 172 an einer Ringschulter 180 des Formstücks 144 **(Fig. 9)** abgestützt. Letzteres besteht aus einem elastischen Kunststoff und hat ein federndes Rastorgan 182 mit einem Rastvorsprung 184, der bei der Dosiseinstellung in eine axiale Längsverzahnung 220 (Fig. 7) des Teils 148 eingreift und bei der Verdrehung des Teils 148 Klickgeräusche verursacht. Diese ermöglichen es blinden Patienten, durch Zählen der Klickgeräusche die gewünschte Dosis einzustellen. Außerdem hält der Rastvorsprung 184 nach der Dosiseinstellung durch Eingriff in die Längsverzahnung 220 das Teil 148 in der eingestellten Lage fest, wirkt also wie eine Kupplung (K3 in Fig. 18), die im Verlauf einer Injektion gelöst wird.

Die Lupe 42 ist in Fig. 9 nur schematisch dargestellt. Sie wird von unten in eine Ausnehmung 186 des Formstücks 144 eingesetzt. Dies ist symbolisch durch eine strichpunktierte Linie 188 angedeutet.

Mit 190 ist in Fig. 9 eine Rückstellfeder für den Einstellknopf 32 bezeichnet. Dies ist eine Drehfeder. Ihr distales Ende 192 ist mit dem Einstellknopf 32 drehfest verbunden, ihr proximales Ende 194 mit dem Formstück 144. Diese Feder 190 dreht nach einer Injektion den Einstellknopf 32 wieder in seine Nullstellung zurück, in der durch die Lupe 42 die Dosis "0" ablesbar ist.

Der Einstellknopf 32 hat auf seiner Innenseite eine Axialverzahnung (splines) 196, welche mit einer entsprechenden Axialverzahnung 198 des Teils 150 der Zustellhülse 151 zusammenarbeitet. (Fig. 9 ist in einem größeren Maßstab gezeichnet als Fig. 7). Eine Öffnung 199 (Fig. 11) am distalen Ende des Einstellknopfs 32 wird durch einen Deckel 200 (Fig. 9 und 35) verschlossen.

**Fig.10** zeigt das Formstück 144 im Längsschnitt. Sein rechter Teil 202 befindet sich praktisch vollständig im Inneren des distalen Gehäuseteils 36. Letzteres hat eine seitliche Aussparung 204 (Fig. 7), und durch diese ragt der Clip 40 nach außen und sichert dabei das Formstück 144 gegen Verdrehung. Längsrippen 203 dienen zur reibungsarmen seitlichen Führung der Feder 172, die in Fig. 7 dargestellt ist.

**Fig.11** zeigt den Einstellknopf 32 im Längsschnitt. Seine innere Axialverzahnung 196 erstreckt sich über etwa zwei Drittel der Gesamtlänge dieses Teils. Dieses hat also am distalen Ende einen kurzen Bereich 206, der keine Axialverzahnung hat, und es hat einen längeren proximalen Bereich 208, wo sich ebenfalls keine Axialverzahnung befindet. Auf seiner Außenseite hat der Einstellknopf 32 einen Ringwulst 210 zum Eingriff in eine entsprechende Ringnut 212 (Fig. 6) des distalen Gehäuseteils 36. In diese Ringnut 212 wird er bei der Montage eingeclipst. Ferner hat er einen Ringraum 211, welcher die Rückstellfeder 190 aufnimmt, die in Fig. 9 dargestellt ist.

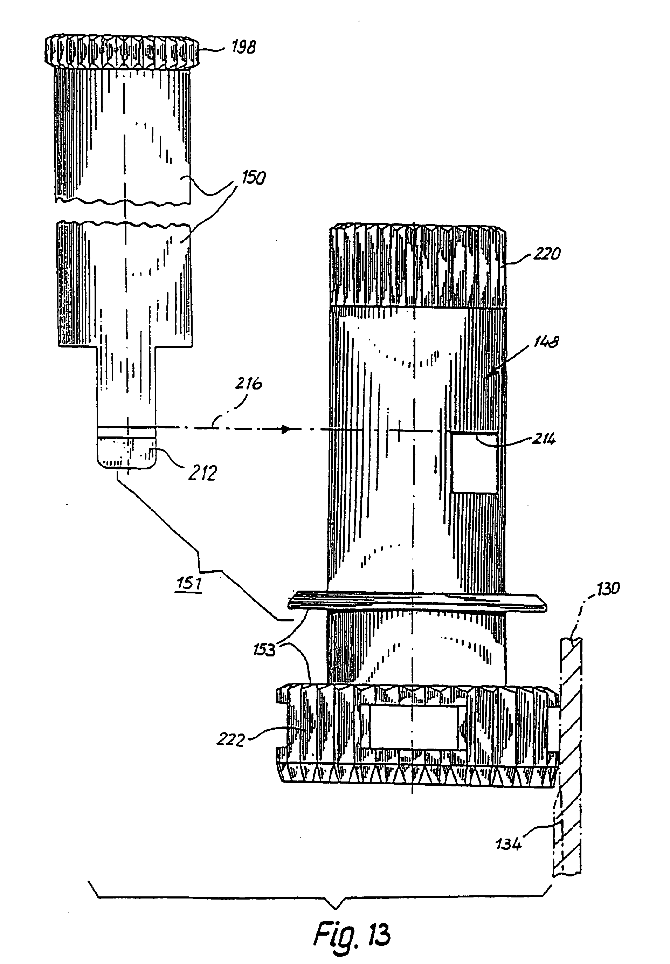

**Fig.12** zeigt das Teil 150 der Zustellhülse 151. Dieses hat am proximalen Ende zwei radial federnde Haken 212 (Fig. 7) zum formschlüssigen Eingriff in entsprechende Ausnehmungen 214 des Teils 148 der Zustellhülse. Dieser Eingriff ist in Fig.13 durch eine strichpunktierte Linie 216 symbolisiert.

Wie man aus den Fig. 11 und 12 gut erkennt, ist die äußere Axialverzahnung 198 des Teils 150 zum formschlüssigen Eingriff in die innere Axialverzahnung 196 des Einstellknopfs 32 ausgebildet. Befindet sich die äußere Axialverzahnung 198 im Eingriff mit der inneren Axialverzahnung 196, so kann durch Drehung des Einstellknopfs 32 das Teil 150, und mit ihm das Teil 148, verdreht werden, d.h. eine Dosiseinstellung ist möglich.

Befindet sich die äußere Axialverzahnung 198 im Bereich 206, so kann der Einstellknopf 32 kein Drehmoment auf das Teil 150 übertragen. Dies ist die Freilaufstellung des Einstellknopfs 32, die bei Fig. 1 und 3 ausführlich erläutert wurde.

Befindet sich die äußere Axialverzahnung 198 im Bereich 208, so kann der Einstellknopf 32 ebenfalls kein Drehmoment auf das Teil 150 übertragen. Dies ist die Stellung nach einer Injektion, und in dieser Stellung wird der Einstellknopf 32 durch die Rückstellfeder 190 (Fig. 9) in seine Nullstellung zurückgedreht, damit die nächste Dosiseinstellung wieder bei der Stellung "0" beginnen kann.

Wie **Fig. 13** besonders deutlich zeigt, hat das Teil 148 an seinem distalen Ende eine äußere Axialverzahnung 220, welche im gespannten Zustand des Geräts 30 mit dem Rastvorsprung 184 (Fig. 10) zusammenwirkt, um bei der Dosiseinstellung klickende Geräusche zu erzeugen und eine einmal eingestellte Dosis "festzuhalten", d.h. eine unbeabsichtigte Verstellung dieser Dosis zu verhindern.

Ferner hat das Teil 148 an seinem proximalen Ende eine äußere Axialverzahnung 222. Im gespannten Zustand des Geräts befindet sich diese Axialverzahnung 222 nicht im Eingriff mit der inneren Axialverzahnung 134 des Innenrohrs 130 (Fig. 6), so dass die Teile 148 und 150, welche zusammen die Zustellhülse 151 bilden, frei verdrehbar sind, um die Dosis einstellen zu können. Dieser Zustand ist vergrößert in Fig. 17 dargestellt (Kupplung K4 geöffnet; alternativ könnte bei Fig. 17 die Kupplung K4 auch geschlossen sein, um eine Verdrehung der Zustellhülse 151 durch den Patienten zu blockieren).

Bei einer Injektion verschiebt sich das Teil 148 (zusammen mit dem Teil 150) in proximaler Richtung, und dabei kommt seine Axialverzahnung 222 in Eingriff mit der axialen Innenverzahnung 134 des Innenrohres 130, das in Fig. 13 nur sehr schematisch angedeutet ist. Diese Lage ist z.B. in Fig. 24 dargestellt (Kupplung K4 geschlossen).

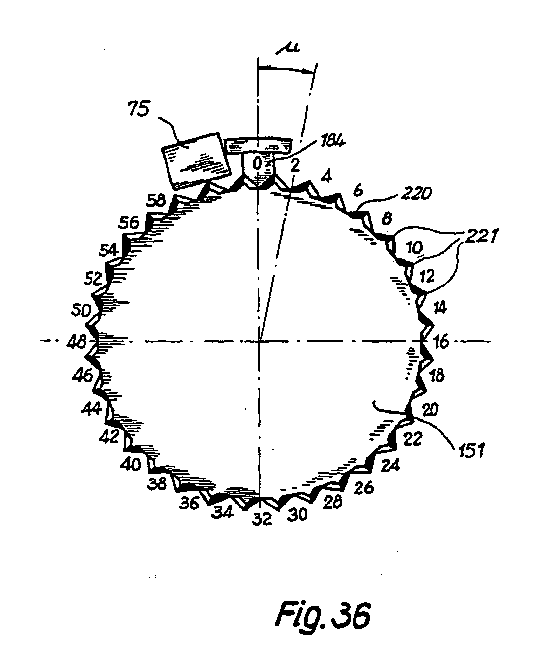

Sobald die axiale Innenverzahnung 134 in die axiale Außenverzahnung 222 eingreift, wird die Zustellhülse 151 an einer Drehung relativ zum Innenrohr 130 gehindert, d.h. die eingestellte Injektionsdosis kann sich während des Injektionsvorgangs nicht verändern. Die Zahl der Zähne der axialen Verzahnung 220 stimmt überein mit der Zahl der Zähne der axialen Verzahnung 222. Beim vorliegenden Ausführungsbeispiel sind es je zweiunddreißig Zähne, um insgesamt dreißig Dosiseinstellungen von 0 bis 58 Einheiten zu ermöglichen, vgl. Fig. 2A und Fig. 36. (Zwei Zähne werden nicht für die Dosiseinstellung verwendet, vgl. Fig. 36 und die zugehörigen Erläuterungen.)

**Flg. 14** zeigt auseinandergezogen das vordere Adapterteil 116 und das hintere Adapterteil 122 in vergrößertem Maßstab und in raumbildlicher Darstellung. Das vordere Adapterteil 116 hat eine Aussparung 224 mit Vorsprüngen 226, welche zusammen zur Verbindung mit der bereits erläuterten diagonalen Nut 120 (Fig. 5) des Kartuschenhalters 80 dienen, um mittels des - umgedrehten - Kartuschenhalters 80 eine Drehung des vorderen Adapterteils 116 zu ermöglichen. Eine solche Verdrehung kann in ungünstigen Fällen beim Kartuschenwechsel notwendig werden.

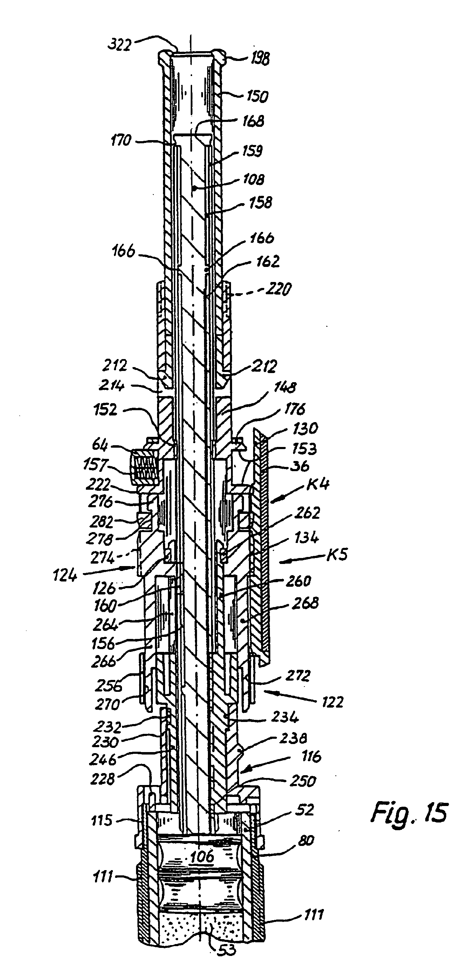

Das vordere Adapterteil 116 hat ferner zwei axiale Kunststoffedem 228, welche gemäß Fig. 15 im montierten Zustand gegen die Kartusche 52 anliegen und diese mit einer Kraft in proximaler Richtung beaufschlagen, vgl. auch Fig. 20.

Ferner hat das vordere Adapterteil 116 eine federnde Zunge 230, die sich in axialer Richtung erstreckt und an deren freiem Ende auf der radial inneren Seite ein Rastglied 232 (mit dreieckförmigem Querschnitt) vorgesehen ist, das mit entsprechenden Rastvorsprüngen 234 des hinteren Adapterteils 122 zusammenwirkt und mit diesen eine Rutschkupplung bildet, die beim Kartuschenwechsel wirksam wird und verhindert, dass der Patient beim Anschrauben des Kartuschenhalters 80 an das vordere Adapterteil 116 ein zu großes Drehmoment auf das vordere Adapterteil 116 erzeugt und dieses dadurch beschädigt. Wird nämlich vom Patienten ein zu hohes Drehmoment in Richtung des Pfeiles 236 (Fig. 14) erzeugt, so rutscht das Rastglied 232 über die Rastvorsprünge 234, und das vordere Adapterteil 116 dreht sich relativ zum hinteren Adapterteil 122, so dass das Injektionsgerät nicht beschädigt werden kann.

Bei der Montage werden die beiden Adapterteile 116, 122 dadurch miteinander verrastet, dass ein Ringwulst 238 des vorderen Adapterteils 116 in eine Ringnut 240 des hinteren Adapterteils 122 eingerastet wird, wie das Fig. 15 zeigt. Die Ringnut 240 befindet sich an zwei axialen Vorsprüngen 252, 254.

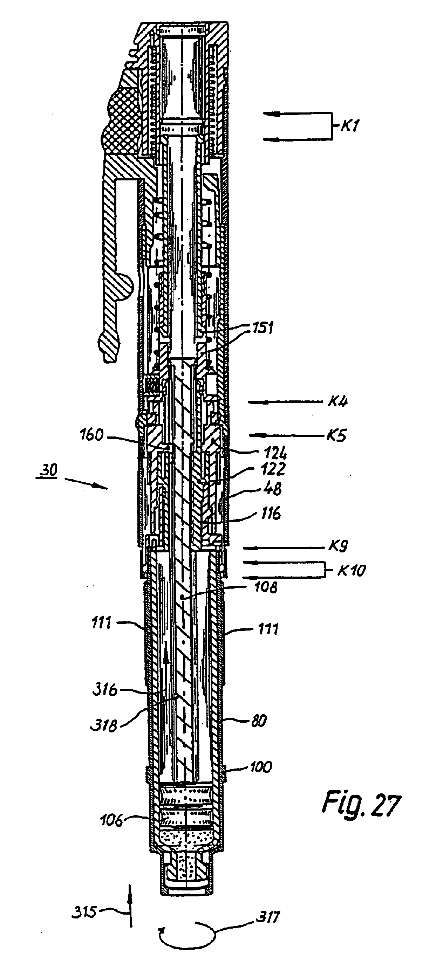

Das vordere Adapterteil 116 hat auch ein radial nach innen ragendes federndes Rastglied 242, das zum Einrasten in die Aussparung 118 (Fig. 5) des am Kartuschenhalter 80 vorgesehenen Außengewindes 114 dient. Das Rastglied 242 rastet nach einem Kartuschenwechsel in diese Aussparung 118 ein. Wenn eine alte Kartusche 52 entfernt werden soll, wird das vordere Adapterteil 116 zunächst durch Drehen des Kartuschenhalters 80 in Richtung eines Pfeiles 237 (Fig. 14) mitgedreht, weil durch das Rastglied 242 ein Drehmoment vom Kartuschenhalter 80 auf das Adapterteil 116 übertragen wird. Diese Drehbewegung dient zur Rückstellung des Stößels 108, wie das nachfolgend bei den Fig. 27 bis 29 beschrieben wird. Erst wenn der Stößel 108 voll zurückgestellt, also in distaler Richtung bis zu einem Anschlag (K2 in Fig. 29) verschoben, ist, rastet das Rastglied 242 aus der Aussparung 118 aus, und der Kartuschenhalter 80 wird vom vorderen Adapterteil 116 abgeschraubt, so dass die Kartusche 52 ausgetauscht werden kann. Auf diese Weise wird sichergestellt, dass bei einem Kartuschenwechsel automatisch der Stößel 108 in distaler Richtung bis zu einem Anschlag (K2 in Fig. 29) verschoben wird. In dieser Stellung wird der Stößel 108 dann durch ein Rastglied 320 verrastet, vgl. Fig. 28 und 29.

Wie Fig. 14 zeigt, befinden sich die Rastzähne 234 auf einem rohrförmigen Abschnitt 246 des hinteren Adapterteils 122. Dieser Abschnitt 246 hat an seinem proximalen Bereich einen radial federnden Abschnitt 248, der auf seiner radial inneren Seite mit Rastzähnen 250 versehen ist. Diese dienen zum Eingriff in die in Fig. 8 dargestellte Mikroverzahnung 162, und die Rastzähne 250 sind deshalb im wesentlichen komplementär zur Mikroverzahnung 162 ausgebildet, vgl. Fig. 15. Sie bilden mit dieser eine Rutschkupplung, d.h. wenn zwischen den Rastzähnen 250 und der Mikroverzahnung 162 eine Verschiebekraft auftritt, die eine vorgegebene Größe überschreitet, rutschen die Rastzähne 250 über die Zähne der Mikroverzahnung 162. Dies ist so in der Endphase einer Injektion, ebenso beim Auswechseln einer Kartusche 52, weil dabei der Stößel 108 in seine distale Endlage zurückgestellt werden muss, vgl. die Fig. 27, 28 und 29.

Die axialen Vorsprünge 252, 254 ragen in proximaler Richtung, und ihre Aufgabe ist es unter anderem, ständig einen vorgegebenen Abstand zwischen dem vorderen Adapterteil 116 und dem hinteren Adapterteil 122 aufrechtzuerhalten und das vordere Adapterteil 116 drehbar zu lagern. Die radial äußere Seite des hinteren Adapterteils 122 ist mit einer axialen Außenverzahnung 256 versehen, welche komplementär zur axialen Innenverzahnung 134 des Innenrohres 130 ausgebildet ist und in diese eingreift, solange sich das Adapterteil 122 im Innenrohr 130 befindet.

Wie die Fig. 14 und 15 zeigen, hat das hintere Adapterteil 122 auch auf seiner distalen Seite einen rohrartigen Fortsatz 260, der in seinem distalen Endbereich mit einer Ringnut 262 versehen ist, welche den in Fig. 6 dargestellten Anschlagring 126 aufnimmt. Der rohrartige Fortsatz 260 hat gemäß Fig. 15 eine axial verlaufende Durchbrechung 264, durch welche der Vorsprung 160 (vgl. Fig. 6) des Führungsteils 124 in die eine Längsnut 156 des Stößels 108 ragt und letzteren dadurch drehfest, aber axial verschiebbar, mit dem Führungsteil 124 verbindet.

Gemäß Fig. 6 hat das Führungsteil 124 zwei axiale Vorsprünge 266, 268, welche einander diametral gegenüberliegen und in proximaler Richtung ragen. Wegen des Ringwulsts 238 (Fig. 14) am vorderen Adapterteil 116 nimmt der Innendurchmesser der Vorsprünge 266, 268 in deren proximalem Bereich 266', 268' zu. Die Vorsprünge 266, 268 werden axial geführt durch zwei zu ihnen etwa komplementäre Führungsöffnungen 272, 270 des hinteren Adapterteils 122, d.h. zwischen Adapterteil 122 und Führungsteil 124 ist keine relative Drehbewegung möglich, wohl aber eine axiale Verschiebung. Letztere ist in der einen Richtung begrenzt durch den Anschlagring 126, vgl. Fig. 15, und in der anderen Richtung durch das Anstoßen der axialen Vorsprünge 266, 268 am vorderen Adapterteil 116, vgl. Fig. 25, oder durch das Anstoßen des Führungsteils 124 am hinteren Adapterteil 122. Das Führungsteil 124 ist auf seiner Peripherie mit einer axialen Außenverzahnung 274 versehen, welche bei einer Injektion in der axialen Innenverzahnung 134 des Innenrohres 130 geführt ist. Da das Führungsteil 124 drehfest mit dem Adapterteil 122 sowie mit dem Stößel 108 verbunden ist, wird die Drehstellung dieser Teile durch die Drehstellung des Führungsteils 124 bestimmt.

Wenn Injektionen stattfinden, ist das Führungsteil 124 ständig drehfest, aber axial verschiebbar, mit dem Innenrohr 130 verbunden. Beim Auswechseln einer Kartusche wird diese drehfeste Verbindung aufgehoben, vgl. Fig. 26 - 32, und das Führungsteil 124, sowie die mit ihm drehfest verbundenen Teile 108 und 122 können sich dann relativ zum Gehäuse 36 drehen, während die Zustellhülse 151 über ihre Außenverzahnung 222 in Eingriff mit dem Innenrohr 130 steht und deshalb nicht gedreht werden kann. Dies ermöglicht eine einfache Rückstellung des Stößels 108, wie das nachfolgend beschrieben wird.

Auf seiner distalen Seite hat das Führungsteil 124 einen rohrartigen Fortsatz 276, auf dessen Außenseite gemäß Fig. 15 eine Ringnut 278 vorgesehen ist.

Gemäß Fig. 7 ist der proximale Teil 148 der Zustellhülse 151 auf seiner proximalen Seite mit einer Aussparung 280 versehen, an der vier radial nach innen ragende Rastabschnitte 282 vorgesehen sind, welche bei der Montage in die Ringnut 278 des Führungsteil 124 eingeclipst werden und dadurch die Verstellhülse 151 drehbar, aber axial unverschiebbar, mit dem Führungsteil 124 verbinden.

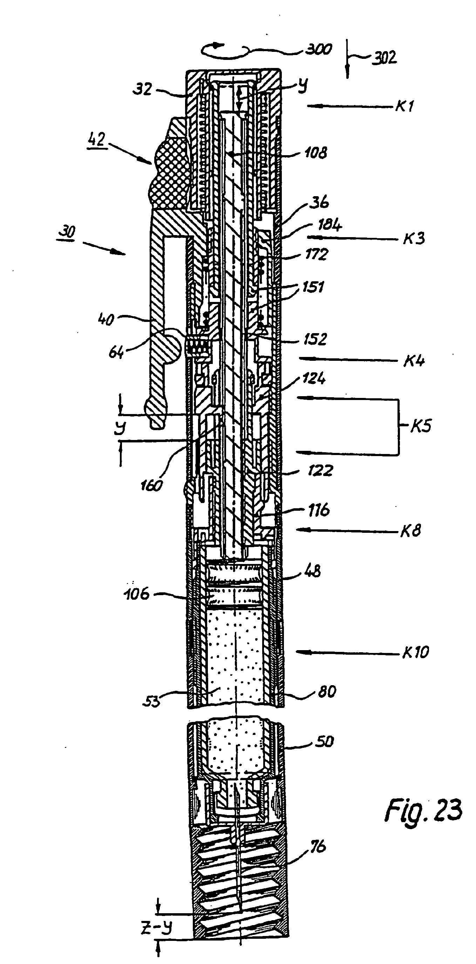

Da das Teil 148 der Zustellhülse 151 über sein Innengewinde 152 in Eingriff mit dem Außengewinde 159 des Stößels 108 steht, und da letzterer durch den Vorsprung 160 des Führungsteils 124 gegen Verdrehung gesichert ist, bewirkt eine Verdrehung der Teile 150, 148 eine axiale Verschiebung des Stößels 108, der mit letzterem über die Zähne 250 (Fig. 14) verbundenen beiden Adapterteile 116, 122, und damit auch des Karpulenhalters 80, d.h. bei der Dosiseinstellung werden alle diese Teile zusammen um einen Weg verschoben, der der gewünschten Einstelldosis entspricht. Dies wird nachfolgend anhand von Fig. 23 näher erläutert.

Die **Fig. 16 und 17** zeigen, was geschieht, wenn der Patient versucht, das Injektionsgerät 30 ohne Nadel zu spannen. In diesem Fall wird die proximale Öffnung 98 des Kartuschenhalters 80 nicht vom Nadelhalter 92 (Fig. 3) überdeckt, und das Teil 56A des Spannknopfs 56 ragt durch diese Öffnung 98 und liegt gegen das proximale Ende der Kartusche 52 an. Wie Fig. 17 zeigt, wird in diesem Fall der Rastknopf 64 nicht so weit in distaler Richtung verschoben, dass er in die Rastöffnung 38 einrasten kann, d.h. ein Spannen des Geräts ist nicht möglich.

In diesem Fall ist in der Stellung gemäß Fig. 16 zwar theoretisch eine Dosiseinstellung möglich, weil der Einstellknopf 32 in Eingriff mit der Dosierhülse 151 steht, aber da keine Nadel vorhanden ist, wirkt die Flüssigkeit 58 in der Kartusche 52 wie ein starrer Körper, der jeder Verschiebung des Stößels 108 einen unüberwindlichen Widerstand entgegensetzt und eine solche Verschiebung verhindert. Eine Dosiseinstellung in dieser Stellung wird also verhindert, und nach dem Abschrauben der Spannkappe 56 geht das Gerät 30 wieder zurück in seine entspannte Stellung gemäß Fig. 4, so dass der Patient gezwungen wird, eine Nadel 76 anzuschrauben, damit er das Gerät 30 spannen, anschließend eine Dosis einstellen, und danach injizieren kann.

Die vorliegende Erfindung macht Gebrauch vom Zusammenwirken verschiedener Kupplungselemente, teilweise in Form von positionsabhängigen Kupplungen, teilweise in Form von kraftabhängigen Kupplungen, und von einer Kupplung, die zwei Elemente drehfest miteinander kuppelt, aber eine axiale Verschiebung zwischen ihnen erlaubt. Fig. 18 zeigt diese Kupplungen in einer schematischen Übersicht.

Zwischen der äußeren Axialverzahnung 198 (Fig. 12) der Zustellhülse 151 und der axialen Innenverzahnung 196 (Fig. 11) des Einstellknopfs 32 besteht eine positionsabhängige Kupplung K1, deren Arbeitsweise bereits beschrieben wurde. Sie ermöglicht eine automatische Rückstellung des Einstellknopfs 72 auf '0" im Verlauf einer Injektion, und sie deaktiviert den Einstellknopf 32, wenn sich das Gerät in seiner Spannstellung gemäß Fig. 3 befindet.

Eine kraft- und lageabhängige Rastkupplung K2 ist vorgesehen zwischen dem distalen Ende des Stößels 108 und dem distalen Ende der Zustellhülse 151. Diese Kupplung K2 wird nachfolgend anhand von Fig. 28 und 29 näher beschrieben. Sie hat eine Funktion beim Austausch der Kartusche 52.

Eine kraft- und lageabhängige Rastkupplung K3 ist vorgesehen zwischen dem gehäusefesten Rastglied 184 und der axialen Außenverzahnung 220 der Zustellhülse 151. Diese Kupplung K3 ist in Eingriff, wenn sich das Gerät in seiner Spannstellung befindet und dient zum Speichem der gewünschten Dosiseinstellung. Während einer Injektion wird K3 geöffnet, ohne dass die gespeicherte Information verlorengehen kann, weil dann die Funktion der Kupplung K3 von einer Kupplung K4 nahtlos übernommen wird. (In Fig. 18 ist das Rastglied 184 nur mit strichpunktierten Linien angedeutet, um das Verständnis zu erleichtern.)

Die Kupplung K4 ist eine positionsabhängige Kupplung, und sie ist vorgesehen zwischen der axialen Außenverzahnung 222 am proximalen Ende der Zustellhülse 151 und der axialen Innenverzahnung 134 des Innenrohres 130. Die Kupplung K4 ist geöffnet, solange sich das Injektionsgerät 30 in seiner Spannstellung befindet, so dass dort die Zustellhülse 151 zwecks Dosiseinstellung verdreht werden kann.

Direkt nach dem Beginn einer Injektion und während des ganzen Verlaufs einer Injektion wird die Kupplung K4 geschlossen, vgl. Fig. 15, d.h. die Zustellhülse 151 ist dann unverdrehbar, aber axial verschiebbar, im Innenrohr 130 geführt. Die Kupplung K4 ist auch geschlossen, wenn eine Kartusche 52 ausgetauscht wird.

Eine positionsabhängige Kupplung K5 ist vorgesehen zwischen der axialen Außenverzahnung 274 des Führungsteils 124 und der axialen Innenverzahnung 134 des Innenrohres 130, ebenso zwischen der axialen Außenverzahnung 256 des hinteren Adapterteils 122 und der axialen Innenverzahnung 134. Diese Kupplung K5 ist ständig geschlossen, solange das Gerät injektionsbereit ist. Sie wird geöffnet, wenn eine neue Kartusche 52 in das Gerät eingesetzt wird, da hierbei die Teile 116, 122 und 124 relativ zur Zustellhülse 151 verdreht werden müssen, um den Stößel 108 in die richtige Lage bringen zu können.

Eine axial verschiebbare Kupplung K6 ist vorgesehen zwischen den Vorsprüngen 266, 268 des Führungsteils 124 und dem hinteren Adapterteil 122. Diese Kupplung ermöglicht die Dosiseinstellung, wie das nachfolgend bei Fig. 23 beschrieben wird.

Eine Rutschkupplung K7 ist vorgesehen zwischen dem vorderen Adapterteil 116 und dem hinteren Adapterteil 122. Diese ist bei Fig. 14 näher beschrieben und wird gebildet von der federnden Rastklinke 232 des vorderen Adapterteils 116 und den Rastzähnen 234 des hinteren Adapterteils 122. Diese Rutschkupplung K7 tritt ggf. beim Kartuschenwechsel in Funktion.

Eine kraftabhängige Rutschkupplung K8 ist vorgesehen zwischen dem hinteren Adapterteil 122 und dem Stößel 108. Sie wird gebildet von der Mikroverzahnung 162 (Fig. 8) des Stößels 108 und der entsprechenden Verzahnung 250 (Fig. 14) am hinteren Adapterteil 122. Sie tritt im Verlauf einer Injektion in Funktion, um das Auspressen von Injektionsflüssigkeit 53 aus der Kartusche 52 zu ermöglichen, ebenso beim Auswechseln einer Kartusche 52.

Eine drehmomentenabhängige Kupplung K9 ist vorgesehen zwischen dem Kartuschenhalter 80 und dem vorderen Adapterteil 116. Sie wird gebildet von der Aussparung 118 (Fig. 5) des Außengewindes 114 und dem radial nach innen ragenden Teil 242 (Fig. 14) des vorderen Adapterteils 116. Diese Kupplung K9 tritt in Funktion, wenn eine Kartusche 52 ausgewechselt wird und stellt sicher, dass beim Kartuschenwechsel der Stößel 108 in die richtige Lage gebracht wird.

Eine lageabhängige Kupplung K10 ist vorgesehen zwischen den Längsrippen 111 des Kartuschenhalters 80 und der axialen Innenverzahnung 112 des mittleren Gehäuseteils 48. Die Kupplung K10 ist in Eingriff, solange Injektionen stattfinden, und sie verhindert, dass beim Wechsel der Injektionsnadel 76 der Kartuschenhalter 80 relativ zum vorderen Adapterteil 116 verdreht wird. Die Kupplung K10 wird beim Kartuschenwechsel automatisch geöffnet, da dann der Kartuschenhalter 80 vom vorderen Adapterteil 116 abgeschraubt werden muss.

Das Zusammenwirken der Kupplungen K1 bis K10 wird aus der Beschreibung der nachfolgenden Figuren ersichtlich. Z.B. sind in Fig. 15 die Kupplungen K4 und K5 geschlossen, und die Kupplung K3 ist geöffnet.

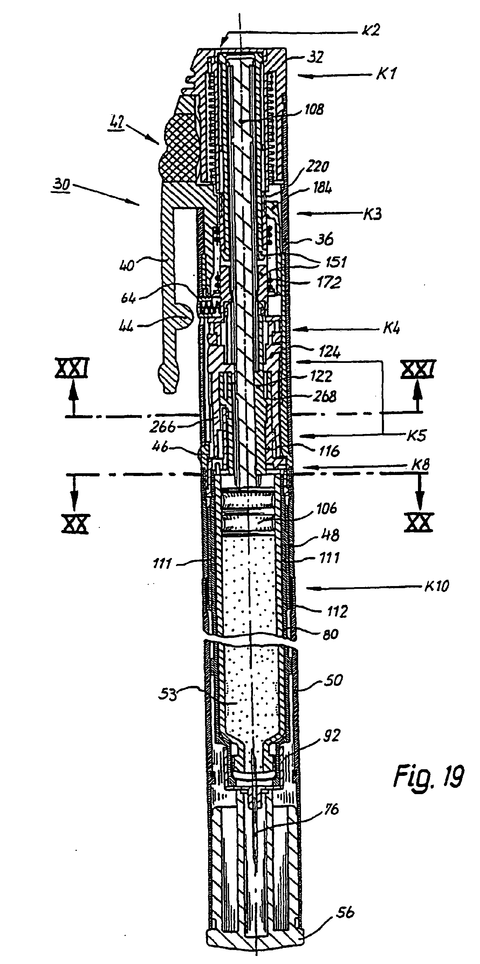

**Fig. 19** zeigt nochmals das Injektionsgerät 30 in seiner Stellung gemäß Fig. 3, aber in einem durchgehenden Längsschnitt. Die Bezeichnung der Teile stimmt mit den vorhergehenden Figuren überein, so dass hierauf Bezug genommen werden kann. Man erkennt, dass beim Spannen die Vorsprünge 266, 268 gegen das vordere Adapterteil 116 anliegen, d.h. die Teile 124, 122, 116 sind - nach einer Injektion - teleskopartig vollständig zusammengeschoben, ebenso wie in den Fig. 16 und 17. Deshalb können sie die Spannkraft von der Spannkappe 56 direkt auf die Feder 172 übertragen und diese so weit zusammenpressen, bis das Rastglied 64 in die Rastausnehmung 38 einrastet.

Die Kupplung K1 ist geöffnet, d.h. eine Dosiseinstellung ist nicht möglich.

Die Kupplung K2 ist geöffnet, d.h. der Stößel 108 ist nicht mit dem Teil 150 verrastet.

Die Kupplung K3 ist aktiviert, d.h. das Rastelement 184 greift in die Längsverzahnung 220 ein.

Die Kupplung K4 ist geöffnet, d.h. die Zustellhülse 151 ist frei verdrehbar.

Die Kupplung K5 ist geschlossen, d.h. das Führungsteil 124 sowie das hintere Adapterteil 122 sind relativ zum Gehäuse 36 nicht verdrehbar.

Die Kupplung K8 ist im Eingriff, d.h. die beiden Adapterteile 116, 122 müssen den axialen Bewegungen des Stößels 108 folgen.

Die Kupplung K10 ist im Eingriff, d.h. die Längsrippen 111 des Kartuschenhalters 80 werden von der Innenverzahnung 112 des mittleren Gehäuseteils 48 geführt, und dadurch wird der Kartuschenhalter 80 gegen Verdrehung gesichert.

**Fig. 20** zeigt im wesentlichen eine Draufsicht auf die Innenseite des vorderen Adapterteils 116 in seinem montierten Zustand. Auf die Erläuterungen zu Fig. 14 wird der Kürze halber verwiesen. Man erkennt insbesondere die Ausnehmung 224, welche zum Eingriff mit dem steckschlüsselartigen Ende 120 (Fig. 5) des Kartuschenhalters 80 ausgebildet ist. Die Ausnehmung 98 des Kartuschenhalters 80 hat einen Durchmesser, der es gestattet, den Stößel 108 durch sie durchzuschieben.

**Fig. 21** zeigt, wie die Teile 252, 254, 266 und 268 interdigital ineinandergreifen. Die Teile 266, 268 dienen, wie bereits beschrieben, dazu, die Teile 122, 124 drehfest miteinander zu verbinden, sie aber axial relativ zueinander verschiebbar zu machen, wie das aus Fig. 6 besonders deutlich hervorgeht.

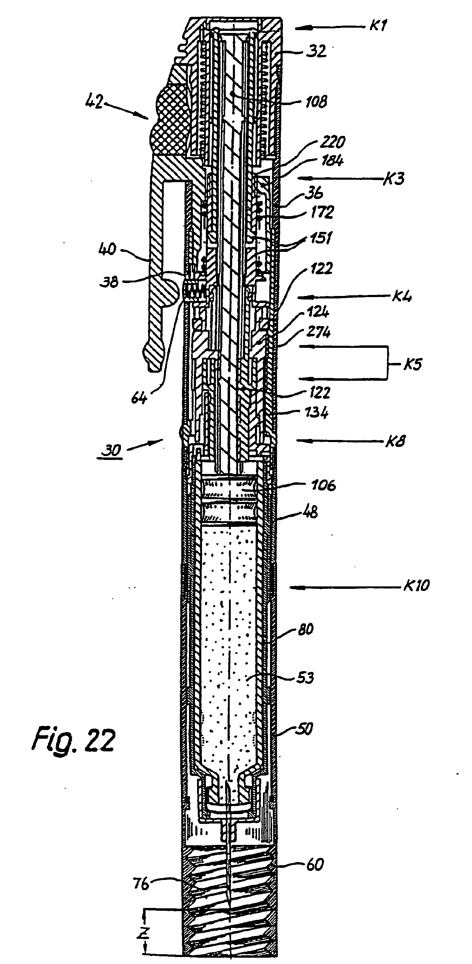

**Fig. 22** zeigt das Injektionsgerät in der Stellung gemäß Fig. 2 und im Längsschnitt. Zur Vorbereitung einer Injektion hat der Patient die Spannkappe 56 (Fig. 2) aus dem Gewinde 60 herausgeschraubt, und die gespannte Feder 172 hat die inneren Teile des Geräts um ca. 2 mm in proximaler Richtung verschoben, wodurch der Rastknopf 64 gegen das proximale Ende der Ausnehmung 38 anliegt.

Dadurch befindet sich nun die Kupplung K1 im Eingriff, d.h. die Zustellhülse 151 kann durch Drehen des Einstellknopfs 32 verdreht werden, um die gewünschte Injektionsdosis einzustellen.

Die Kupplung K3 befindet sich weiterhin im Eingriff, d.h. die Rastklinke 184 greift in die Längsverzahnung 220 ein.

Die Kupplung K4 befindet sich nicht im Eingriff, d.h. die Zustellhülse ist zur Dosiseinstellung relativ zum Gehäuse verdrehbar.

Die Kupplung K5 befindet sich im Eingriff, d.h. das Führungsteil 124, das hintere Adapterteil 122, und der Stößel 108 können sich relativ zum Gehäuse 36 nicht verdrehen.

Die Kupplung K8 ist im Eingriff, d.h. das hintere Adapterteil 122 ist mit dem Stößel 108 und dem Kartuschenhalter 80 auch in axialer Richtung gekuppelt, so dass sich diese Teile in axialer Richtung nur gemeinsam bewegen können.

Die Kupplung K10 ist ebenfalls im Eingriff.

Die proximale Spitze der Injektionsnadel 76 hat in diesem Fall den Abstand Z vom proximalen Ende des Gehäuseteils 50. Dies ist der maximale Abstand, und durch die Dosiseinstellung wird er verkleinert, wie das nachfolgend bei Fig. 23 beschrieben wird.

Bei **Fig. 23** wird der Einstellknopf 32 im Uhrzeigersinn verdreht (Pfeil 300 in Fig. 23), gesehen in Richtung eines Pfeiles 302, also gesehen in proximaler Richtung.

Da die Kupplung K1 im Eingriff ist, wird hierdurch die Zustellhülse 151 verdreht, welche durch das Rastglied 64 in ihrer axialen Stellung festgehalten wird. Die Zustellhülse 151 schraubt mit ihrem Innengewinde 152 den Stößel 108 in proximaler Richtung, da dieser durch den Eingriff des Teils 160 des Führungsglieds 124 an einer Drehung gehindert ist.

Die Kupplung K4 ist geöffnet, d.h. die Zustellhülse 151 kann sich frei drehen.

Die Kupplung K5 ist geschlossen, d.h. das Führungsglied 124 und das hintere Adapterteil 122 sind an einer Drehung relativ zum Gehäuseteil 36 gehindert.

Bei einer Verschiebung in proximaler Richtung nimmt der Stößel 108 über die Kupplung K8 (vgl. die Beschreibung zu Fig. 18) das hintere Adapterteil 124 mit. Dieses verschiebt das mit ihm verbundene vordere Adapterteil 116 und mit letzterem den Kartuschenhalter 80 in proximaler Richtung um einen Weg Y, der der zu injizierenden Dosis entspricht. Der Abstand des proximalen Endes der Nadel 76 vom proximalen Ende des Gehäuseteils 50 verringert sich dadurch in Fig. 23 von Z (Fig. 22) auf (Z - Y). Nach wie vor ist die Nadel 76 für den Patienten nicht sichtbar.

Nach der Einstellung der zu injizierenden Dosis ist das Injektionsgerät 30 nun fertig für eine Injektion. Die eingestellte Dosis Y bleibt gespeichert, weil die Kupplung K3 eine Drehung der Zustellhülse 151 verhindert.

Bei einer Injektion setzt der Patient das Gerät auf den Körperteil, wo eine Injektion vorgenommen werden soll, z.B. auf das Gesäß, und drückt dann gemäß Fig. 24 mit einer symbolisch angedeuteten Kraft F auf den Clip 40, so dass dieser nach innen ausgelenkt wird und mit seinem Vorsprung 44 den Rastknopf 64 nach innen drückt, so dass die gespannte Injektionsfeder 172 die Zustellhülse 151 in proximaler Richtung verschiebt. Dabei gleitet der Rastknopf 64 in der axialen Längsnut 136 (Fig. 6) des Innenrohres 130.

Hierbei bleibt die Kupplung K1 zunächst geschlossen. Die Kupplung K3 öffnet sich, da die axiale Verzahnung 220 aus dem Rastorgan 184 herausgleitet. Während dieses Herausgleitens gleitet die Zustellhülse 151 mit ihrer Außenverzahnung 222 (Fig. 13) in die Innenverzahnung 134 (Fig. 6) des Innenrohres 130 und wird dadurch ohne Unterbrechung an einer Drehung gehindert, so dass die vom Patienten eingestellte Dosis unverändert gespeichert bleibt. Die Zustellhülse 151 bewirkt über ihr Innengewinde 152 eine axiale Verschiebung des Stößels 108 in proximaler Richtung. Da dieser über die Kupplung K8 mit dem hinteren Adapterteil 122 verbunden ist, wird dieses ebenfalls in axialer Richtung bewegt, und mit ihm das vordere Adapterteil 116 und der Kartuschenhalter 80, so dass gemäß Fig. 24 die Nadel 76 in den Patienten eingestochen wird. Dies ist also der Vorgang des Einstechens der Nadel 76.

Die Kupplung K5 bleibt hierbei geschlossen, d.h. das Führungsglied 124 kann sich relativ zum Gehäuseteil 36 nicht verdrehen. Das Führungsglied 124 steht in direkter Antriebsverbindung mit der Zustellhülse 151, so dass auch das Führungsglied 124 in proximaler Richtung verschoben wird, sich dabei aber nicht drehen kann und auch - über sein Eingriffsglied 160 - eine Drehung des Stößels 108 verhindert.

Der Kartuschenhalter 80 wird beim Einstechvorgang so weit in proximaler Richtung verschoben, bis sein Ringbund 100 gegen den Dämpfungsring 102 stößt, der an der Ringschulter 104 abgestützt ist. Dies beendet die Bewegung des Kartuschenhalters 80 in proximaler Richtung. Die Kupplung K10 bleibt dabei ständig in Eingriff, wobei in Fig. 24 die axiale Innenverzahnung 112 des mittleren Gehäuseteils 48 nur noch mit dem distalen Endbereich der Längsrippen 111 in Eingriff steht, wie in Fig. 24 dargestellt.

**Fig. 25** zeigt den weiteren Verlauf einer Injektion. Da der Ringbund 100 im Anschlag gegen den Dämpfungsring 102 ist, kann sich der Kartuschenhalter 80 nicht mehr weiter in proximaler Richtung bewegen, und hierdurch wird die Kupplung K8 gelöst, d.h. die Zähne 250 des Teils 248 (Fig. 14) gleiten jetzt über die Mikroverzahnung 162 (Fig. 8) des Stößels 108, so dass sich dieser - unabhängig vom Kartuschenhalter 80 - weiter in proximaler Richtung bewegt und dabei den Kolben 106 in der Kartusche 52 um den eingestellten Weg Y verschiebt und so aus der Nadel 76 die vom Patienten eingestellte Dosis des Medikaments 53 auspresst, was in Fig. 25 bei 304 symbolisch angedeutet ist.

Bei dieser Bewegung ändern das vordere Adapterteil 116 und das hintere Adapterteil 122 ihre axiale Lage im Gehäuseteil 48 nicht mehr, bleiben also stehen, während sich die Zustellhülse 151 und das mit ihr axial verbundene Führungsteil 124 um den eingestellten Weg Y weiterbewegen. Da der Stößel 108 über das Gewinde 152 der Zustellhülse 151 mit dieser axial verbunden ist, bewegt sich der Stößel 108 ebenfalls um den Weg Y in proximaler Richtung und verschiebt dabei den Kolben 106, da sich die Kartusche 52 nicht mehr in proximaler Richtung weiterbewegen kann.

Bei ihrer axialen Bewegung gleitet die Zustellhülse 151 mit ihrer axialen Außenverzahnung 198 aus der axialen Innenverzahnung 196 des Einstellknopfs 32 heraus, d.h. die Kupplung K1 wird geöffnet. Deshalb kann die Drehfeder 190 den Einstellknopf 32 in seine Nullstellung zurückdrehen, was in Fig. 25 durch den Drehpfeil 310 symbolisiert ist.

Bei der Schlussphase einer Injektion werden also die inneren Teile des Injektionsgeräts 30 um einen bestimmten Weg Y teleskopartig zusammengeschoben, um den sie zuvor beim Einstellen der Dosis (Fig. 23) auseinanderbewegt worden waren.

Die Kupplungen K4 und K5 sind hierbei im Eingriff. Die Kupplung K8 ist während der Schlussphase einer Injektion geöffnet, kommt aber danach sofort wieder in Eingriff. Die Kupplung K10 bleibt ständig in Eingriff.

Die Kupplung K5 wird hier dadurch gebildet, dass das distale Ende der Außenverzahnung 274 (Fig. 6) des Führungsteils 124 mit dem proximalen Endbereich der Innenverzahnung 134 in Eingriff steht. Über das Führungsteil 124 wird der Stößel 108 gegen Verdrehung gesichert (Teil 160 des Führungsteils 124).

Nach der Injektion zieht der Patient die Nadel 76 aus dem Gewebe, wechselt sie ggf. gegen eine neue Nadel aus, und schraubt dann die Spannkappe 56 auf, so dass das Gerät 30 wieder den Zustand gemäß Fig. 3 und 19 einnimmt. In diesem Zustand kann das Gerät z.B. in einem Etui oder einer Handtasche transportiert werden. Sofern mit dem Gerät Insulin injiziert wird, ist eine gekühlte Lagerung (Kühlschrank) wünschenswert.

Wenn der Inhalt 53 einer Kartusche 52 verbraucht ist, sieht dies der Patient durch das Sichtfenster 54 (Fig. 5) oder 54A (Fig. 34). Der Stößel 108 hat gemäß Fig. 15 Anschläge 166, von denen in diesem Fall einer gegen das Führungsglied 160 des Führungsteil 124 stößt, was die Dosiseinstellung blockiert und dem Patienten anzeigt, dass er/sie jetzt die Kartusche 52 wechseln muss. Der Patient kann in diesem Fall nur noch die Restmenge an Wirkstoff dosieren, die sich in der Kartusche 52 befindet.

Bei einem Kartuschenwechsel wird gemäß **Fig. 26** von dem nicht gespannten Injektionsgerät 30 das proximale Gehäuseteil 50 abgeschraubt. Dies ist in Fig. 23 durch einen Drehpfeil 312 symbolisiert.

Da nun der Anschlag 102, 104 fehlt, verschiebt sich unter der Wirkung der Feder 172 das vordere Adapterteil 116 bis zum Anschlag gegen den distalseitigen Innenrand 314 (Fig. 5) der axialen Innenverzahnung 112 des mittleren Gehäuseteils 48. Dabei öffnet sich die Kupplung K5, während die Kupplung K4 im Eingriff bleibt. Ebenso öffnet sich die Kupplung K10, d.h. der Kartuschenhalter 80 kann nun relativ zum mittleren Gehäuseteil 48 verdreht werden. Auch die Kupplung K1 bleibt geöffnet, d.h. eine Dosiseinstellung ist nicht möglich.

**Fig. 27** zeigt das Injektionsgerät nach dem Abschrauben des proximalen Gehäuseteils 50. Der Kartuschenhalter 80 kann, wie bereits erläutert, jetzt in Richtung eines Drehpfeils 317 verdreht werden, also entgegen dem Uhrzeigersinn, gesehen in Richtung eines Pfeils 315.

Hierbei bleibt zunächst die Kupplung K9 (Fig. 18) geschlossen, d.h. die federnde Zunge 242 (Fig. 14) ragt in die Aussparung 118 (Fig. 5) des Außengewindes 114. Die Drehbewegung in Richtung des Pfeiles 317 wird deshalb auf das vordere Adapterteil 116 und von diesem auf das hintere Adapterteil 122 und das Führungsteil 124 übertragen. Letzteres dreht über sein Eingriffsglied 160 den Stößel 108, da die Kupplung K5 geöffnet ist. Da die Kupplung K4 im Eingriff ist, wird der Stößel 108 in Richtung des Pfeiles 316 in distaler Richtung geschraubt. Dabei rutschen die Zähne 250 des hinteren Adapterteils 122 (Fig. 14) über die Mikroverzahnung 162 (Fig. 8) des Stößels 108. Letzterer wird solange in distaler Richtung geschraubt, bis sein Anschlag 318 gegen das Eingriffsglied 160 des Führungsteils 124 stößt. Dieser Zustand ist in Fig. 28 dargestellt.

Jetzt kann sich der Stößel 108 nicht mehr in distaler Richtung weiterbewegen, und folglich können sich die Teile 116, 122 und 124 nicht mehr drehen, so dass jetzt die Kupplung K9 gelöst und der Kartuschenhalter 80 vom vorderen Adapterteil 116 abgeschraubt wird.

Diesen Zustand zeigen die **Fig. 28 und 29.** Der Stößel 108 ist dabei im Inneren des Gehäuses 36, 48 geparkt, und sein distales Ende 168 steht über die dortige Ringnut 170 in Rasteingriff mit einem radial nach innen ragenden Kragen 322 des Teiles 150. Die Rastkupplung K2 ist also in diesem Zustand in Eingriff und hält den Stößel 108 fest.

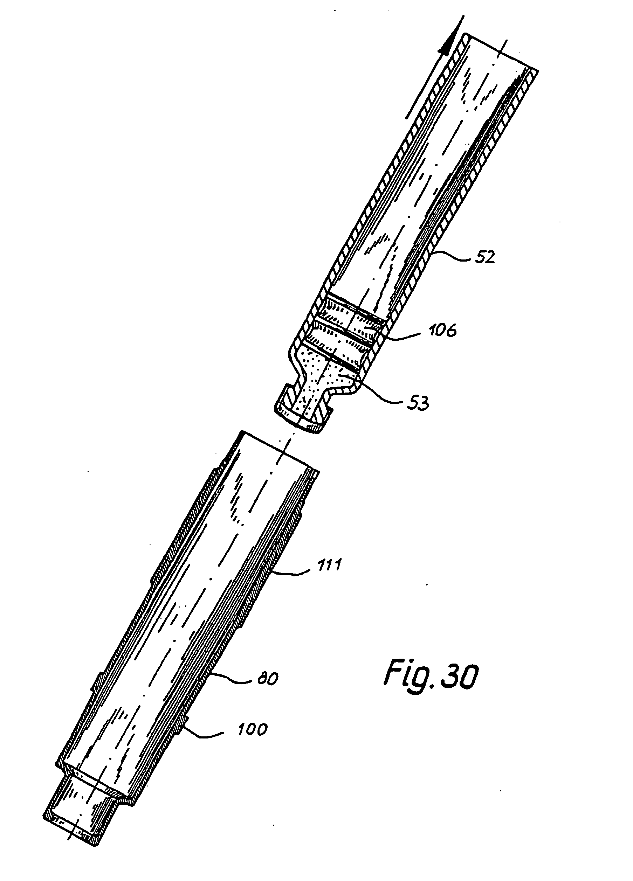

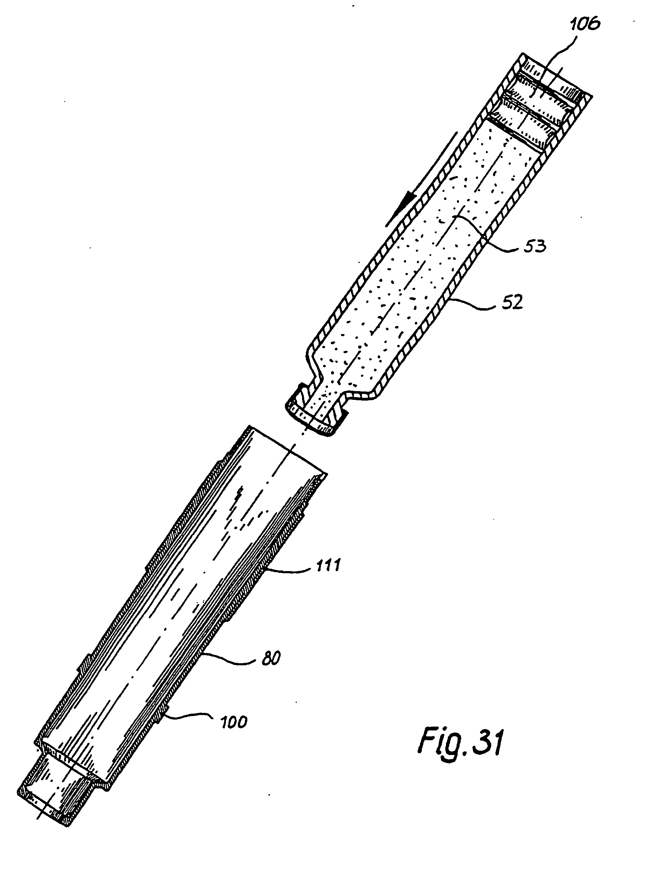

Gemäß **Fig. 30** wird nun dem abgeschraubten Kartuschenhalter 80 die leere Kartusche 52 entnommen, und gemäß **Fig. 31** wird in den Kartuschenhalter 80 eine volle Kartusche 52 eingesetzt.

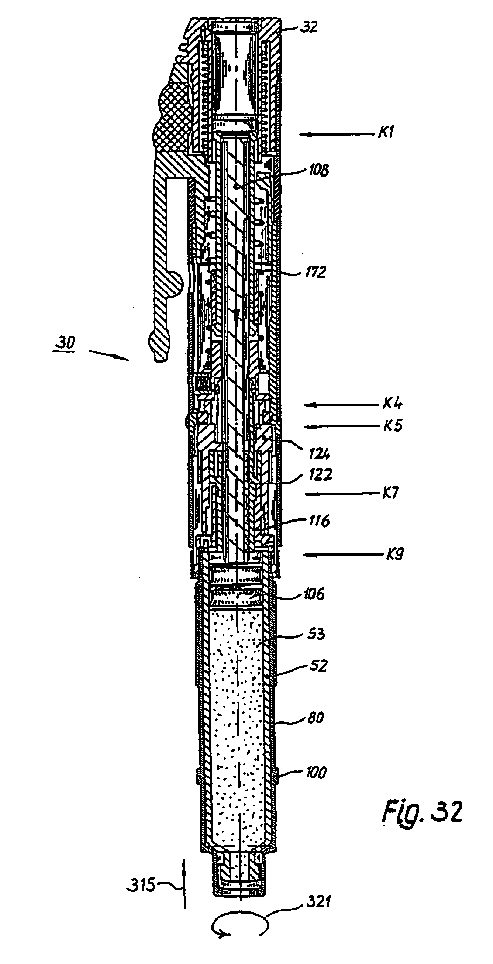

Gemäß **Fig. 32** wird der Kartuschenhalter 80 mit der neuen Kartusche 52 wieder an das vordere Adapterteil 116 angeschraubt. Gesehen in distaler Richtung 315 wird hierzu der Kartuschenhalter 80 im Uhrzeigersinn verdreht. Da die Rastkupplung K2 gemäß Fig. 29 geschlossen ist, kann sich der Stößel 108 zunächst nicht in proximaler Richtung bewegen, wodurch auch eine Drehbewegung des Führungsteils 124, des hinteren Adapterteils 122 und des vorderen Adapterteils 116 blockiert ist, so dass der Kartuschenhalter 80 voll in das vordere Adapterteil 116 eingeschraubt wird, bis dort die Rastkupplung K9 zum Eingriff kommt. Erst dann wird durch das vom Benutzer erzeugte Drehmoment die Rastkupplung K2 gelöst, und der Stößel 108 wird in proximaler Richtung so weit verschoben, bis er sich sanft gegen den Kolben 106 anlegt.

Da die Kartusche 52 flüssigkeitsdicht verschlossen ist, kann sich der Kolben 106 in ihr nicht verschieben. Versucht der Benutzer mit Gewalt, den Kartuschenhalter 80 in Richtung des Drehpfeils 321 (Fig. 32) weiterzudrehen, so tritt die Kupplung K7 in Aktion, d.h. die Rastklinke 232 (Fig. 14) gleitet über die Rastzähne 234. Der Benutzer kann also in diesem Fall das Injektionsgerät 30 auch durch Anwendung von Gewalt nicht beschädigen, weil bei Überschreiten eines vorgegebenen Drehmoments in dieser Drehrichtung die Kupplung K7 bewirkt, dass sich das vordere Adapterteil 116 relativ zum hinteren Adapterteil 122 frei drehen kann. Die Kupplung K7 wirkt also in der soeben beschriebenen Richtung als Rutschkupplung. In der Gegenrichtung ist dies nicht erforderlich, weil in dieser Richtung der Kartuschenhalter 80 vom vorderen Adapterteil 116 abgeschraubt wird.

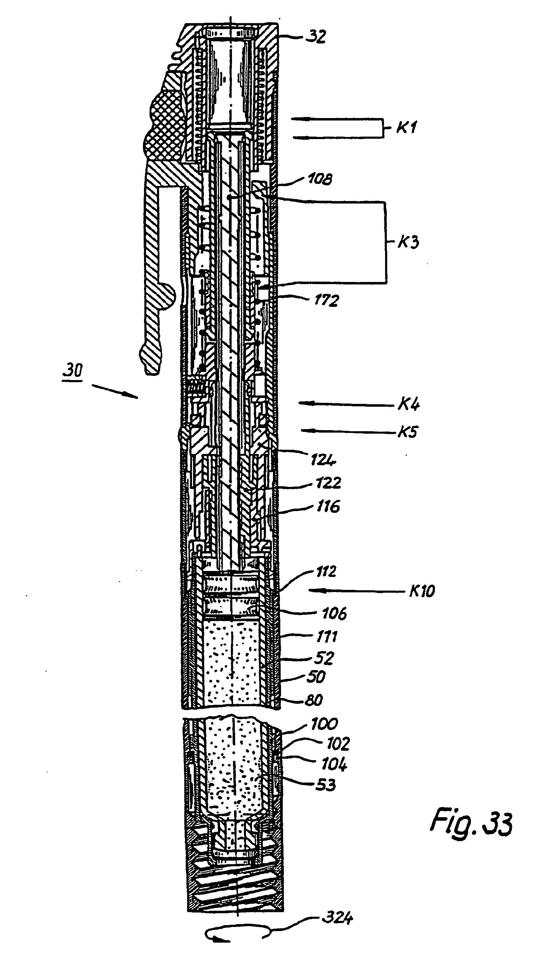

Die Rutschkupplung K7 verhindert, dass der Patient durch ein zu großes Drehmoment den Gummikolben 106 elastisch zusammenpresst. Dies hätte nämlich zur Folge, dass in Fig. 33 nach dem Anschrauben der Nadel 76 (Fig. 3) aus dieser Nadel Injektionsflüssigkeit 53 herausspritzen würde, was unerwünscht ist. Die Kupplung K7 verhindert dies.

Gemäß **Fig. 33** wird nun noch das proximale Gehäuseteil 50 angeschraubt (Drehpfeil 324), wodurch die Kupplung K10 geschlossen wird, und das Injektionsgerät 30 befindet sich dann in einer Position analog Fig. 25 und ist wieder voll gebrauchsfertig, d.h. es sind keine weiteren Einstell- oder Testvorgänge notwendig. Sollten sich in der Kartusche 52 Luftblasen befinden, so können diese dadurch entfernt werden, dass eine kleine Injektionsdosis nach oben in die Luft gespritzt wird. Dies wird den Patienten beim Unterricht im Krankenhaus gezeigt.

Durch das Anschrauben des Gehäuseteils 50 wird der Kartuschenhalter 80 in distaler Richtung verschoben, weil der Ringbund 104 des Gehäuseteils 50 den Ringwulst 100 des Kartuschenhalters 80 in distaler Richtung verschiebt. Die Kupplung K10 kommt in Eingriff, und der Kartuschenhalter 80 wird mit seinen Längsrippen 111 wieder unverdrehbar in der Innenverzahnung 112 (Fig. 5) geführt. Ebenso kommt die Kupplung K5 wieder in Eingriff, und die Kupplung K4 bleibt in Eingriff, was eine Verstellung des Stößels 108 blockiert. Die Kupplung K1 bleibt weiterhin außer Eingriff, so dass die Dosiseinstellung deaktiviert ist. Der Patient kann also die Lage des Stößels 108 in dieser Stellung nicht beeinflussen, wenn er am Gerät herumspielt. Die Kupplung K3 ist in dieser Position nicht in Eingriff. Die Teile 124, 122, 116 sind nach wie vor teleskopartig zusammengeschoben.

Das Gerät kann nach Einsetzen einer Injektionsnadel 76 durch Einschrauben der Spannkappe 56 gespannt werden und befindet sich dann in einem transportfähigen Zustand.

**Fig. 34** zeigt eine Variante, bei der als Sichtfenster eine Vielzahl kleiner Löcher 54A verwendet wird. Dies ist im Rahmen der Erfindung die bevorzugte Lösung, weil der Patient durch diese Löcher 54A nicht hindurchgreifen und deshalb den Ablauf einer Injektion nicht mit den Fingern bremsen kann.

**Fig. 35** zeigt das Injektionsgerät der Fig. 34 von oben, aber gegenüber Fig. 34 in einem größeren Maßstab. **Fig. 36** zeigt eine schematische Darstellung der axialen Verzahnung 220 der Zustellhülse 151 (Fig. 7), und des Rastglieds 184 (Fig. 8), welches bei der Dosiseinstellung in diese Verzahnung 220 fedemd eingreift. Die Dosiseinstellungen von Null bis "58" sind beispielhaft angegeben.

Die Verzahnung 220 hat insgesamt zweiunddreißig Zähne 221, so dass der Winkel µ zwischen zwei benachbarten Zähnen 360°/32 =11,5° beträgt. Wegen des Anschlags 75 (Fig. 9) werden zwei Zähne nicht für die Dosiseinstellung verwendet.

Die Zahl von zweiunddreißig Zähnen wird in gleicher Weise bei den Längsverzahnungen 112, 134, 196, 198, 220, 222, 256 und 274 verwendet, damit die Teile des Injektionsgeräts 30 problemlos ineinander und auseinander gleiten können. Diese Verzahnungen haben aus diesem Grund auch die gleiche Winkellage relativ zueinander, also keine "Phasenverschiebung".

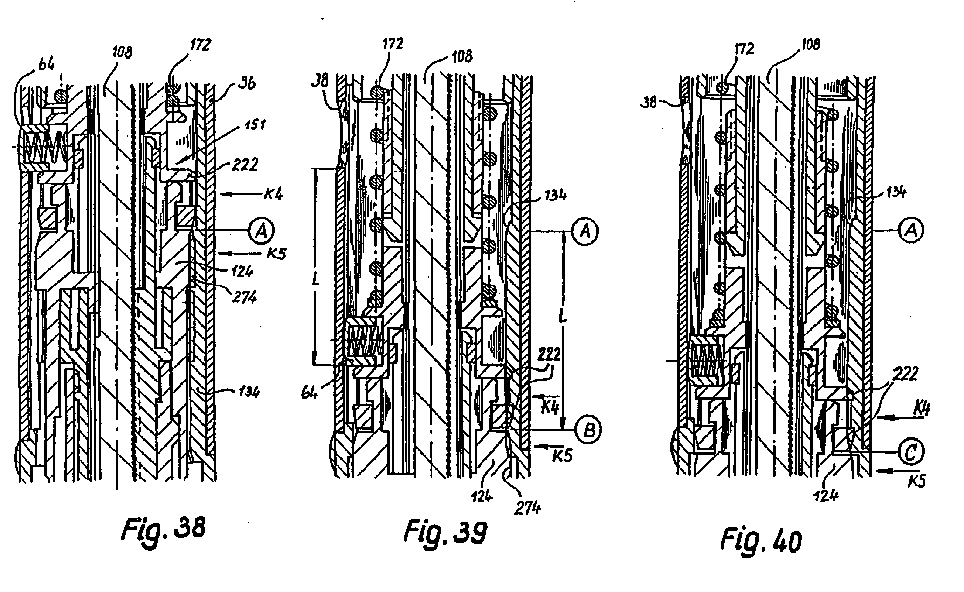

Zur Arbeitsweise noch folgendes: Der Weg L (Fig. 25 und 39), den das Rastglied 64 nach seiner Auslösung bei einer Injektion zurücklegt, ist immer gleich.

Auch die Einstichtiefe U (Fig. 25) der Nadel 76 bleibt im Normalfall unverändert. (Selbstverständlich kann eine Stichtiefenverstellung verwendet werden, um die Stichtiefe an den Patienten anzupassen).

Unterschiedlich ist beim Weg L der Anteil Y, der für die Verschiebung des Kolbens 106 verwendet wird. Dieser Anteil Y wird vor der Injektion dadurch festgelegt, dass die Teile 124 und 122 bei der Dosiseinstellung um diesen Weg Y auseinandergeschoben werden, vgl. Fig. 23, wodurch die Nadel 76 - bereits vor der Injektion - um diesen Weg Y in proximaler Richtung verschoben wird. Der Weg der Nadel 76 bei einer Injektion ist also um den Weg Y kürzer als L, wie bei den Fig. 22 und 23 erläutert.

Wie nachfolgend anhand der Fig. 39 und 40 erläutert wird hat die axiale Innenverzahnung 134 (Fig. 6) etwa die Länge L, weil sie das Führungsteil 124 während des gesamten Wegs L gegen Drehung sichern muss. Am Ende einer Injektion steht das Führungsteil 124 mit seiner Verzahnung 274 gerade noch mit deren distalem Ende in Eingriff mit der Innenverzahnung 134, wie Fig. 25 und 39 zeigen, und vor Beginn einer Injektion hat man gemäß Fig. 22 und 38 die Situation, dass die axiale Verzahnung 222 der Zustellhülse 151 ganz knapp vor dem Eingriff in die Innenverzahnung 134 steht, weshalb die Verzahnung 274 des unmittelbar benachbarten Führungsteils 124 voll mit der Innenverzahnung 134 in Eingriff steht, vgl. Fig. 22 und 38.

Auf diese Weise ergibt sich eine kurze Bauweise des Injektionsgeräts bei sehr sicherer Funktion, und der Austausch einer Kartusche 52 wird erleichtert.

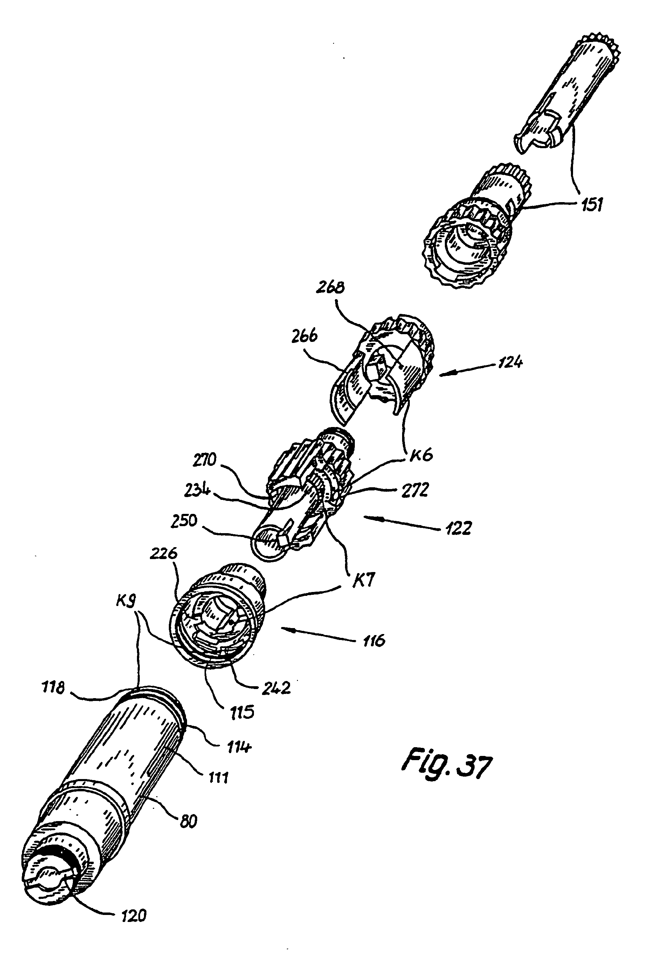

**Fig. 37** veranschaulicht nochmals die Kupplungen, die beim Kartuschenwechsel tätig werden.

Zwischen dem Behälter 80 und dem vorderen Adapterteil 116 befindet sich eine lösbare Verbindung (Außengewinde 114, Innengewinde 115), die ggf. auch als Bajonettverschluss oder dgl. ausgebildet werden könnte.

In dieser lösbaren Verbindung befindet sich die Kupplung K9 (Teile 118, 242). Sie bewirkt beim Öffnen des Behälters 80, dass der Stößel 108 in die Raststellung gemäß Fig. 29 verschoben wird.

Zwischen dem vorderen Adapterteil 116 und dem hinteren Adapterteil 122 befindet sich die Kupplung K7 (Rastglied 232 - in Fig. 14 sichtbar - und Rastzähne 234). Die Kupplung K7 sichert beim Schließen des Behälters 80, dass der Stößel 108 nur sanft in Anlage gegen den Kolben 106 gebracht wird und diesen nicht elastisch verformt.

Zwischen dem hinteren Adapterteil 122 und dem Führungsteil 124 befindet sich die Kupplung K6 (Vorsprünge 266, 268 und Ausnehmungen 270, 272). Sie ermöglicht eine Verstellung des axialen Abstands zwischen den Teilen 122, 124 und die Übertragung eines Drehmoments zwischen ihnen.

Ggf. kann man die Kupplungen K7 und K9 kombinieren, z.B. bei Verwendung eines Bajonettverschlusses für den Behälter 80 (an Stelle der Gewinde 114, 115).

Wenn die Kupplung K9 defekt ist, kann der Benutzer das proximale Ende 120 des Behälters 80 als Steckschlüssel verwenden, um das vordere Adapterteil 116 durch Eingriff in dessen Teil 226 zu drehen.

Die Fig. 38 bis 40 zeigen synoptisch verschiedene mögliche Stellungen der Kupplungen K4 und K5.

Bei Fig. 38 ist das Gerät gespannt und - bei der Dosiseinstellung Null - auslösebereit. Die Kupplung K4 ist nicht in Eingriff. Die Kupplung K5 ist im Eingriff. Die Grenze zwischen Zustellhülse 151 und Führungsteil 124 befindet sich an der Stelle A, nämlich am distalen Ende der Innenverzahnung 134.

Bei Fig. 39 ist die Injektion abgeschlossen. Das Rastglied 64 hat den Weg L zurückgelegt. Die Grenze zwischen den Teilen 151 und 124 hat sich an die Stelle B bewegt, die von der Stelle A den Abstand L hat. Die Stelle B befindet sich oberhalb des unteren Endes der Längsverzahnung 134, d.h. letztere ist länger als L, damit in Fig. 39 die Kupplung K5 geschlossen bleiben kann, d.h. der distale Teil der Längsverzahnung 274 bleibt in Eingriff mit der Innenverzahnung 134. Die Kupplung K4 steht während der Injektion in Eingriff.

**Fig. 40** zeigt den Zustand beim Kartuschenwechsel. Die genannte Grenze zwischen den Teilen 151 und 124 hat sich an die Stelle C verschoben. Die Kupplung K4 ist weiterhin geschlossen, d.h. das Teil 151 kann sich nicht drehen, und die Kupplung K5 ist geöffnet, um das Führungsteil 124 beim Kartuschenwechsel verdrehen zu können, wie bereits ausführlich beschrieben. Der Abstand von A bis C entspricht etwa der axialen Länge der Innenverzahnung 134 und ist größer als L.

Statt der dargestellten Kupplungen können auch andere Arten von Kupplungen verwendet werden, z.B. Kupplungen, welche Magnete verwenden. Falls das Gerät z.B. von einem Mikroprozessor oder Mikrocontroller gesteuert wird, können Kupplungen durch elektrische Energie betätigt werden.

Naturgemäß sind im Rahmen der vorliegenden Erfindung auch vielfache andere Abwandlungen und Modifikationen möglich.

## Patentansprüche

1. Injektionsgerät mit einem Behälter (80) zur Aufnahme einer eine Injektionsflüssigkeit (53) enthaltenden Kartusche (52), an deren proximalem Ende eine Injektionsnadel (76) befestigbar ist,
mit einem in einem Gehäuse angeordneten, zum Auspressen von Injektionsflüssigkeit (53) aus der Kartusche (52) dienenden Stößel (108), welcher ein Außengewinde (159) aufweist, das in einem Innengewinde (152) eines zur Einstellung der Injektionsdosis dienenden Einstellglieds (151) geführt ist,
und welcher in einem Führungsglied (124) axial verschiebbar geführt ist,
**gekennzeichnet durch** eine Antriebsverbindung (232, 234, 266, 268, 270, 272), welche zwischen dem Führungsglied (124) und dem Behälter (80) vorgesehen ist und welche eine Vorrichtung (118, 242; 232, 234) aufweist, die in mindestens einer Drehrichtung das vom Behälter (80) auf das Führungsglied (124) übertragbare Drehmoment begrenzt,
um **durch** Übertragung eines begrenzten Drehmoments vom Behälter (80) auf das Führungsglied (124) nach einem Kartuschenwechsel eine Verstellung des Stößels (108) zur Anlage gegen einen in der Kartusche (52) vorgesehenen Kolben (106) zu ermöglichen.

2. Injektionsgerät nach Anspruch 1, bei welchem die Vorrichtung zur Begrenzung des Drehmoments eine Rutschkupplung (232, 234) aufweist.

3. Injektionsgerät nach Anspruch 1 oder 2, bei welchem das Führungsglied (124) eine Kupplung (K5) zur drehfesten Verbindung mit dem Gehäuse aufweist, und diese Kupplung (K5) lösbar ist, um ein Drehmoment vom Behälter (80) auf das Führungsglied (124) übertragen zu können und letzteres **dadurch** relativ zum Gehäuse zu drehen.

4. Injektionsgerät nach einem der vorhergehenden Ansprüche,
bei welchem im Behälter (80, 116) mindestens ein Federglied (228) vorgesehen ist, welches die Kartusche (52) in proximaler Richtung beaufschlagt.

5. Injektionsgerät nach Anspruch 4, bei welchem das Federglied (228) einstückig mit einem Glied (116) ausgebildet ist, welches nach Art eines Deckels mit dem Behälter (80) verbindbar ist.

6. Injektionsgerät nach einem der vorhergehenden Ansprüche, bei welchem der Behälter (80) im Gehäuse (50, 48, 46, 36) zwischen einer proximalen und einer distalen Stellung verschiebbar ist.

## Claims

1. Injection device comprising a container (80) for reception of a cartridge (52) which contains an injection fluid (53) and on whose proximal end an injection needle (76) can be mounted,
comprising a plunger (108), arranged in a barrel and serving to expel injection fluid (53) out of the cartridge (52), which plunger comprises an external thread (159) that is guided in an internal thread (152) of a setting member (151) serving to set the injection dose, and is guided axially displaceably in a guide member (124),
**characterised by** a drive connection (232, 234, 266, 268, 270, 272) which is provided between the guide member (124) and the container (80) and comprises a device (118, 242; 232, 234) that limits, in at least one rotation direction, the torque transferable from the container (80) to the guide member (124) in order to allow adjustment of the plunger (108) to bear against a piston (106) provided in the cartridge (52) through transfer of a limited torque from the container (80) to the guide member (124) after a change of cartridge.

2. Injection device according to claim 1, wherein the device for limiting the torque comprises a slip coupling (232, 234).

3. Injection device according to claim 1 or 2, wherein the guide member (124) comprises a coupling (K5) for non-rotatable connection to the barrel, and this coupling (K5) is releasable in order to be able to transfer a torque from the container (80) to the guide member (124) and thereby turn the latter relative to the barrel.

4. Injection device according to one of the preceding claims, wherein at least one spring member (228), which acts on the cartridge (52) in the proximal direction, is provided in the container (80, 116).

5. Injection device according to claim 4, wherein the spring member (228) is embodied in one piece with a member (116) which can be connected to the container (80) in the manner of a cover.

6. Injection device according to one of the preceding claims, wherein the container (80) is displaceable in the barrel (50, 48, 46, 36) between a proximal and a distal position.

## Revendications

1. Appareil d'injection muni d'un récipient (80) pour recevoir une cartouche (52) renfermant un liquide d'injection (53) et à l'extrémité proximale de laquelle une aiguille d'injection (76) peut être fixée, comprenant un coulisseau (108), logé dans un boîtier, qui sert à expulser du liquide d'injection hors de la cartouche (52) et qui présente un filetage extérieur (159) guidé dans un filetage intérieur (152) d'un organe de réglage (151) pour le réglage de la dose d'injection, et qui est guidé dans un organe de guidage (124) avec faculté de coulissement axial,
**caractérisé par** une liaison d'entraînement (232, 234, 266, 268, 270, 272) qui est prévue entre l'organe de guidage (124) et le récipient (80) et qui présente un dispositif (118, 242, 232, 234) qui limite, au moins dans un sens de rotation, le couple de rotation pouvant être répercuté par le récipient (80) sur l'organe de guidage (124), afin de permettre par transmission d'un couple de rotation limité du récipient (80) sur l'organe de guidage (124), après le remplacement d'une cartouche, un déplacement du coulisseau (108) en application contre un piston (106) prévu dans la cartouche (52).

2. Appareil d'injection selon la revendication 1, dans lequel le dispositif de limitation du couple de rotation est réalisé sous la forme d'un accouplement patinant (232, 234).

3. Appareil d'injection selon la revendication 1 ou 2, dans lequel l'organe de guidage (124) comporte un accouplement (K5) pour une liaison, sans faculté de rotation, avec le boîtier, et cet accouplement étant amovible, afin de pouvoir transmettre un couple de rotation du récipient (80) sur l'organe de guidage (124) et de ce fait pouvoir tourner ce dernier par rapport au boîtier.

4. Appareil d'injection selon l'une des revendications précédentes, dans lequel est prévu, dans le récipient (80, 116), au moins un élément à ressort (228) qui sollicite la cartouche (52) dans le sens proximal.

5. Appareil d'injection selon la revendication 4, dans lequel l'élément à ressort (228) est réalisé d'une seule pièce avec un élément (116) qui peut être relié au récipient (80) à la manière d'un couvercle.

6. Appareil d'injection selon l'une des revendications précédentes, dans lequel le récipient (80) peut être coulissé dans le boîtier (50, 48, 46, 36) entre une position proximale et une position distale.
